# EUROPEAN PATENT APPLICATION

(11) **EP 2 280 075 A1**
(43) Date of publication of application: **02.02.2011**
(21) Application number: 09166871.5
(22) Date of filing: 30.07.2009
(51) Int. Cl.: C12P 13/00

(54) **Biocatalyst for catalytic hydroamination**

(71) Applicant: BASF SE, 67056 Ludwigshafen (DE)
(72) Inventor: The designation of the inventor has not yet been filed

(57) **Abstract**

The present invention relates to a method for the enzymatic hydroamination of C-C double bonds catalyzed by enzymes structurally and/or functionally related to phenylalanine ammonia lyase (PAL) isolated from microorganisms of Petroselinum crispum, Rhodoturula glutinis and/or functional active derivatives thereof.

## Description

The present invention relates to a method for the enzymatic hydroamination of C-C double bonds catalysed by enzymes structurally and/or functionally related to phenylalanine ammonia lyase (PAL) isolated from microorganisms of Petroselinum crispum, Rhodoturula glutinis and/or functional active derivatives thereof.

### Background of the invention

Industrial application of amines ranges from simple use as solvents, additives for pharmaceuticals, bactericides, flotation auxiliaries, anti-foam agents, corrosion inhibitors, detergents or dyes. Classical methods for their synthesis, either on laboratory or industrial scale, include transformations of alcohols or alkyl halides into amines, reductive amination of carbonyl compounds, aminoalkylation, reduction of amides, nitriles, azides or nitro compounds and last but not least the Ritter reaction.

Although the direct addition of amines to alkenes is thermodynamically feasible (Δ (delta) H° ≈ -52.7 kJ mol⁻¹, Δ(delta) S° ≈ -127.3 J K⁻¹ mol⁻¹ and Δ (delta) G° ≈ -14.7 kJ mol⁻¹ for the addition of ammonia to ethylene), the hydroamination usually requires assistance from a catalyst for the following reasons:
- An electrostatic repulsion between the nitrogen electron pair of the approaching amine and the delta-bond of the electron-rich olefin results in a high activation barrier
- A concerted [2+2] addition of the N-H bond to the alkene is an orbital-symmetry forbidden process and is unfavourable due to the large difference in energy between the carbon-carbon Π (Pi)-bond and the N-H-σ(sigma)-bond
- The equilibrium is shifted towards the starting material as elevated temperatures due to the negative reaction entropy.

Increasing research effort has led to the development of many transition metal based catalyst systems (early transition metals of group 3-5, lanthanides and actinides, and late transition metals of group 8-11) over the last two decades. The addition of amine functionalities to unsaturated carbon-carbon bonds can be performed either in an intramolecular or intermolecular fashion.

However, the hydroamination of alkenes is more difficult compared with that of alkynes because of the lower reactivity and electron density of C-C double bonds. Intermolecular hydroamination of alkenes that are catalysed by late transition metals provide low rates and have limited scope. A particular challenge is the reversal of the regiochemistry to obtain the anti-Markovnikov product. (T. E. Müller, K. C. Hultzsch, M. Yus, F. Foubelo, M. Tada, Chem. Rev. 2008, 10, 3795-3892). No general catalyst has been described combining high catalytic activity and a consistent high level of enantioselectivity for a wide range of substrates, high functional group tolerance and userfriendlyness. Although primary amines are accessible via hydroamination using ammonia equivalents, the direct addition of ammonia under mild conditions has remained elusive. Asymmetric hydroamination using ammonia are highly desirable from atom-economical and cost-efficiency points of view. (K. C. Hultzsch, Adv. Synth. Catal. 2005, 347, 367-391.)

Biocatalysis frequently allows the production of optically active chiral molecules from prochiral precursors, by the use of enzymes giving an alternative to asymmetric chemical synthesis. As enzymes are proteins they are biodegradable, and due to their reactions being largely conducted in water, are consistent with the physiological conditions that they experience in nature. The relatively mild reaction conditions of enzymes require less energy than would typically be required for synthetic chemical reactions which are generally carried out in organic solvents.

A group of enzymes called amino acid ammonia lyases has intrigued organic chemists and enzymologists, since this specific reaction can not be done non-enzymatically.

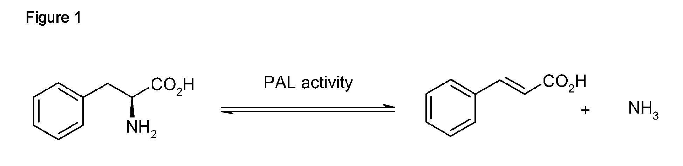

Ammonia lyases in their natural role are involved in the metabolism of amino acids and also play a role in, for instance, the degradation of amino sugars, but only a limited amount of these enzymes have been characterised biochemically. The family of *L*-amino acid ammonia lyases mediate the abstraction of a proton from the β-(beta)-carbon and the removal of the amino group from their substrate amino acids in nature (Figure 1). However, the reverse reaction is unknown in nature. The biosynthesis of phenylalanine follows the well known shikimate pathway. The reverse reaction using trans-cinnamic acid to form L-phenylalanine is not described in any metabolism in plants. (G. M. Kishore, D. M. Shah, Annu. Rev. Biochem. 1988, 57, 627-663.)

However, these enzymes have already been used for industrial production of amino acids. In 1960, a Japanese company Tanabe Seiyaku started the industrial production of (S)-Asp from fumaric acid by immobilised cells of *E*. *coli* containing aspartate ammonia lyase as one of the examples of the industrial use of the enzyme (Y. Asano, Y. Kato, C. Levy, P. Baker, D. Rice, Biocat. Biotrans. 2004, 22, 131-138).

Large-scale fermentation procedures for L-Phe production, using Rhodotorula PAL, have been used by Genex Corporation and Pfizer Corporation. Enantiomerically pure L-tyrosine has been synthesised from the corresponding p-hydroxycinnamic acid by using red yeast (Rhodotorula rubra) resting cells possessing phenylalanine ammonia lyase as biocatalyst. (J.-S. Zhao, S.-K- Yang, Chin. J. Chem. 1995, 13, 241-245.) A procedure for the conversion of trans-cinnamyl methyl ester to L-phenylalanine methyl ester was described using Rhodotorula sp. 70% conversion and e.e. >99% of product were obtained using an organic-aqueous biphasic system. (G. B. D'Cunha, V. Satyanarayan, P. M. Nair, Enzyme Microb. Technol. 1994, 16, 318-322). Acrylic acids substituted with heteroaryl groups at the beta-position were synthesized using phenylalanine ammonia lyase from parsley. The heteroaryl groups were furanyl, thiophenyl, benzofuranyl, and benzothiophenyl and contained the alanyl side chains either at the 2- or 3-positions. (C. Paizs, A. Katona, J. Retey, Chem. Eur. J. 2006, 12, 2739-2744.)

Enantiopure L-phenylalanine and to a lesser extent L-tyrosine can be produced by using high concentrations of ammonia at high pH in the presence of phenylalanine ammonia lyases. Experiments using D- and L-phenylalanine showed that D-phenylalanine does not serve as substrate and is a competitive inhibitor. (K. R. Hanson, Arch. Biochem. Biophys. 1981, 211, 575-588.) This indicates a very high substrate specifity of these enzymes.

Application of a broad range of different ammonia lyases in organic chemical synthesis on an industrial scale has thus far not occurred, which is due to their narrow substrate spectrum.

These enzymes include:
- Histidine Ammonia Lyase HAL
- Phenylalanine Ammonia Lyase PAL
- Tyrosine Ammonia Lyase TAL
- Aspartate Ammonia Lyase AAL
- 3-Methylaspartate Ammonia Lyase MAL
- 3-methylaspartase

It is an object of the present invention to provide an enzymatic catalysed method for the production of aromatic amines. It is a further an object of the invention to provide an enzymatic catalysed method for the addition of amines to the alpha-position of a double bond.

### Summary of the present invention

The above mentioned problem could surprisingly be solved by identifying enzymes which catalyse the hydroamination of a double bond in a side-chain of an aromatic substrate. In particular, phenylalanine ammonia lyases (PAL) isolated from Petroselinum crispum and /or Rhodoturula glutinis have been identified as useful to catalyse the above reaction.

### Detailed description of the invention

According to one aspect of the present invention there is provided a method of hydroaminating a C-C double bond in a side-chain of an aromatic substrate, which method comprises the following steps:
a) contacting the aromatic substrate in the presence of an ammonia compound with
   i) a protein having phenylalanine ammonia lyase activity isolated from Petroselinum crispum and /or Rhodoturula glutinis, ii) a protein having phenylalanine ammonia lyase activity having a sequence identity of at least 70 % to SEQ-ID No. 1 or to SEQ-ID. No. 2,
   iii) a protein coded by a nucleic acid sequence having a sequence identity of at least 70 % to SEQ No. 3 or SEQ No. 4 and/or
   iv) functional equivalents of the proteins according to i), ii) and/or iii), and
b) optionally isolating the hydroamination product from the aromatic substrate,
   wherein the hydroamination product is not L-tyrosine and/or L-phenylalanine.

Proteins having less than 100% sequence identity to SEQ-ID-Nos. 1 or 2 or proteins coded by a nucleic acid having a sequence identity less than 100% to SEQ-ID-Nos.3 or 4 are hereinafter referred to as mutant proteins. Proteins having 100% sequence identity to SEQ-ID-Nos. 1 or 2 or proteins coded by a nucleic acid having a sequence identity of 100% to SEQ-ID-Nos. 3 or 4 are hereinafter referred to as parent proteins.

Mutant proteins according to the present invention having phenylalanine ammonia lyase activity according to ii) preferably include proteins having at least 70 %, more preferably at least 80 %, more preferably at least 90 %, more preferably at least 98 % sequence identity to SEQ-ID No. 1 or to SEQ-ID No. 2. In one embodiment the mutant protein having phenylalanine ammonia lyase activity has 70 to 99, 75 to 98, 80 to 97, 85 to 95, 80 to 90 % sequence identity to the corresponding parent protein having SEQ-ID NOs. 1 or 2.

Mutant proteins according to the present invention having phenylalanine ammonia lyase activity according to iii) preferably include proteins coded by a nucleic acid sequence having a sequence identity of at least 70 %, more preferably at least 80 %, more preferably at least 90 %, more preferably at least 98 % sequence identity to SEQ-ID No. 3 or to SEQ-ID. No. 4. In one embodiment the mutant protein having phenylalanine ammonia lyase activity is coded by a nucleic acid sequence having 70 to 99, 75 to 98, 80 to 97, 85 to 95, 80 to 90 % sequence identity to the nucleic acid sequence SEQ-ID Nos. 3 or 4.

Such deduced mutants may either have substantially the same enzyme characteristics, as for example enzymatic activity, substrate specificity, stereo specificity, stability and/or solubility, as the corresponding non-mutated parent enzyme, or have modified enzyme characteristics, as for example modified, i.e. increased or diminished enzymatic activity, catalytic efficiency, stability, solubility, stereospecificity, and/or modified, i.e. broader or narrower, substrate specificity.

Sequences SEQ-ID Nos. 1 or 2 correspond to the wildtype protein of Petroselinum crispum and /or Rhodoturula glutinis. SEQ-ID-Nos. 3 and 4 correspond to the codon optimised wildtype nucleic acid which has been codon optimised with respect to the expression in Escherichia coli. During the optimisation process the following cis-acting sequences were preferably avoided: internal TATA-boxes, chi-sites, ribosomal entry sites, AT-rich, GC-rich sequences stretches, repeat sequences and/or RNA-secondary structures. Two stop codons were added to ensure efficient termination.

Preferably the modified enzyme activity of the mutated proteins is at least 20 %, preferably at least 50 %, more preferably at least 80 %, more preferably at least 90 %, compared with the protein coded by SEQ-ID-No. 1 or 2 or the protein coded by a nucleic acid sequence SEQ-ID-No. 3 or 4. For example PAL activity may be for example determined spectrophotometrically by following the formation of the hydroamination product from the aromatic substrate at 270 nm. Standard conditions for the measurement of PAL activity may be for example 0.1 M TRIS buffer pH 8.8, 3 mM substrate at 30°C.

The side chain according to the present invention may be for example an alkenyl chain having 2, 3, 4, 5, 6, 7, 8, 9 or 10 carbon atoms. The alkenyl chain may be branched or linear. The alkenyl chain may comprise at least one C-C double bond, preferably one or two double bonds.

Surprisingly, the enzymes specified above are suitable to catalyse the hydroamination of a C-C double bond in a side chain of an aromatic substrate which does not provide the naturally occurring the amino acids and/or derivatives thereof.

Preferably, products of the hydroamination reaction are not selected from the group of naturally occurring amino acids consisting of e.g. L-tyrosine, L-phenylalanine, L-proline, L-hydroxyproline, L-histidine and/or L-tryptophane. More preferably, products of the hydroamination reaction are not selected from the group of naturally occurring amino acids consisting of e.g. L-tyrosine, L-phenylalanine, L-proline, L-hydroxyproline, L-histidine and/or L-tryptophane also including derivatives thereof. For example derivatives of tyrosine, phenylalanine, proline, hydroxyproline, histidine and/or tryptophane include derivatives substituted at the ring system. Substituents may be for example chlorine, fluorine, bromine, cyanide, hydroxyl and/or nitro. Hydroamination reactions excluded from the present invention may lead for example to reaction products like phenylalaninol and/or tyrosinol. Also m-tyrosine may be excluded. Also tyrosine, phenylalanine, proline, hydroxyproline, histidine and/or tryptophane, which are N-methylated may be for example a derivative product excluded from the hydroamination reaction according to this invention. The amino group of the excluded reaction product after the amination may be for example substituted with one or two methyl and/or ethyl groups. In one embodiment the reaction products of the hydroamination reaction excluded from the present invention also include the D-isomer. Preferably, the reaction product is no amino acid ester, amino alcohol and/or aldehyde derived from the above mentioned natural amino acids. More preferably, the reaction product is no amino acid ester, amino alcohol and/or aldehyde. In an alternative embodiment the reaction product is no derivative of tyrosine, phenylalanine, wherein the phenyl ring has been replaced by a heteroycyclic ring, e.g. indole, imidazole, pyrimidine, pyridine, pyrolidine and/or piperidine. Preferably, the reaction product is no pyridinylalanine, pyrimidinylalanine and/or a derivative thereof. In an alternative embodiment the substrate is no dihydro ring analogue of cinnamic acid, i.e. trans-3-(1,4-cyclohexadienyl)-acrylic acid. For example the reaction product is no N-methyl-L-phenylalanine, N-methyl-4-nitro-L-phenylalanine and/or N, N-dimethyl-4-nitro-L-phenylalanine.

Preferably, substrates for the hydroamination reaction are not selected from the group consisting of: trans-2-fluoro-cinnamic acid, trans-3-fluorocinnamic acid, trans-4-fluorocinnamic acid, trans-2,6-difluorocinnamic acid, trans-3,5-difluorocinnamic acid, trans-2,3,4,5,6-pentafluorocinnamic acid, trans-2-chlorocinnamic acid, trans-3-chlorocinnamic acid, trans-4-chlorocinnamic acid, trans-3-(2-pyridyl)-acrylic acid, trans-3-(3-pyridyl)- acrylic acid, trans-3-(4-pyridyl)- acrylic acid, trans-3,5-pyrimidinyl-2-propenoic acid, trans-2,6-dichlorocinnamic acid, trans-3-bromocinnamic acid, trans- 3-cyanocinnamic acid, trans-4-cyanocinnamic acid, trans-3-hydroxycinnamic acid, trans-4-hydroxycinnamic acid, trans-2-nitrocinnamic acid, trans-3-nitrocinnamic acid, trans-3-(1-naphthyl)-acrylic acid, trans-cinnamyl methyl ester, trans-3-furan-2-yl-acrylic acid, trans-3-thiophen-2-yl-acrylic acid, trans-3-furan-3- yl-acrylic acid, trans-3-thiophen-3- yl-acrylic acid, trans-3-benzofuran-2- yl-acrylic acid, trans-3-benzo[b]thiophen-2- yl-acrylic acid, trans-3-benzofuran-3- yl-acrylic acid and/or trans-3-benzo[b]thiophen-3- yl-acrylic acid.

### Preferred substrates

In one embodiment the double bond in a side chain of the aromatic substrate is a non-activated double-bond. Non-activated double bonds are preferably not substituted with a carbon acid group and/or a carbon acid ester group. The carbon acid ester group may be for example a carbon acid methyl ester, ethyl ester and/or propyl ester group.

Preferably the double bond is substituted with a phenyl ring and at least one further C-containing group, wherein the double bond is preferably in trans-position with respect to the aromatic ring. Suitable C-containing groups are selected from the group consisting of methyl, ethyl, n-propyl, iso-propyl, acid amide, nitrile and/or carbonyl.

The C-C double bond in the side chain of the aromatic substrate can be in conjugation to the double bond or not. For example the double bond and the aromatic ring can be separated by a methylene group. Preferably the double bond is conjugated to the aromatic ring. Preferably, the aromatic ring is not substituted with an electron-accepting group, for example a chlorine, bromine and/or fluorine. Preferably the aromatic ring is no heterocyclic group. Preferably, the aromatic group is no 5-membered heteroarylic ring and/or 6-membered heteroarylic ring, e.g. preferably no furanyl, thiophenyl, benzofuranyl, benzothiophenyl, pyridinyl and/or pyrimidinyl group. In one embodiment the aromatic ring is a substituted or unsubstituted phenyl group.

Optionally, the aromatic ring is substituted with an ether and/or hydroxyl group. Preferably, the substituents of the aromatic ring are in trans-position and/or in meta-position to the side-chain having the double bond.

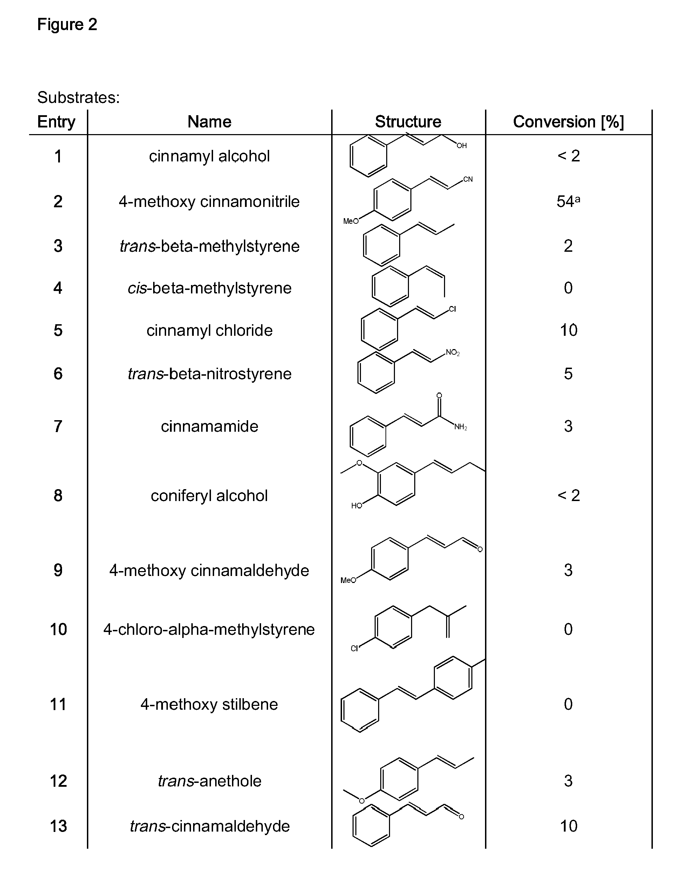

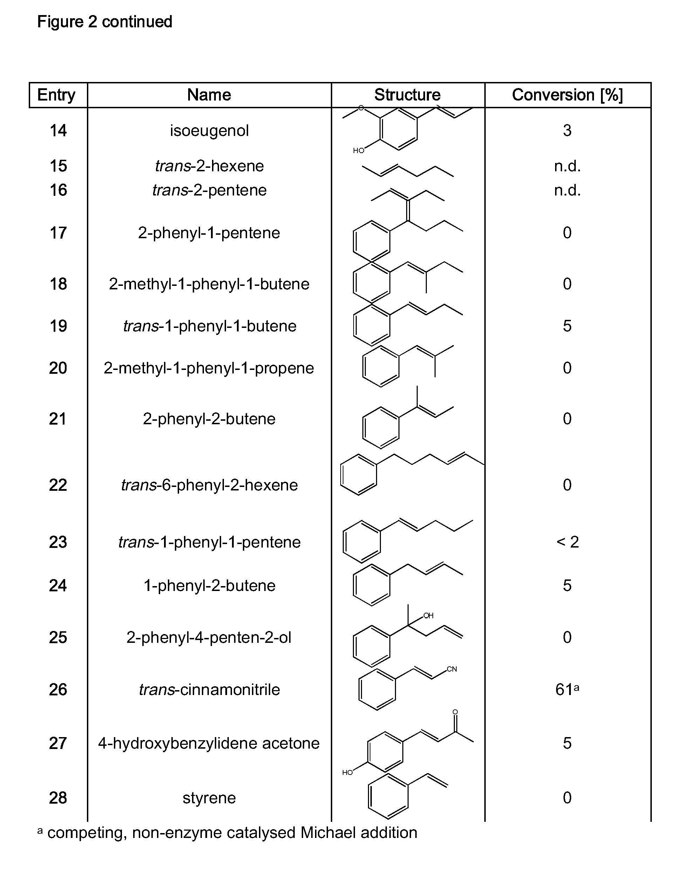

In one embodiment the substrate is selected from the group consisting of trans-beta-methylstyrene, 4-methoxy cinnamaldehyde, 4-methoxy cinnamonitrile, cis-beta-methylstyrene, cinnamyl chloride, trans-beta-nitrostyrene, styrene, cinnamamide, 4-chloro-alpha-methylstyrene, 4-methoxy stilbene, trans-cinnamaldehyde, 2-phenyl-1-pentene, trans-1-phenyl-1-butene, 2-phenyl-4-penten-2-ol, trans-cinnamonitrile, 1-phenyl-2-butene, cinnamyl alcohol, coniferyl alcohol, 4-hydroxybenzylideneacetone, trans-anethole, 2-phenyl-2-butene, 2-methyl-l-phenyl-1-butene, 2-methyl-1-phenyl-1-propene, trans-1-phenyl-1-pentene, trans-6-phenyl-2-hexene, trans-2-hexene, trans-2-pentene and mixtures thereof (cf. Figure 2).

Preferred substrates are cinnamyl chloride, trans-beta-nitrostyrene, isoeugenol, trans-anethole, 4-methoxy cinnamaldehyde, cinnamaldehyde, 4-hydroxybenzylidene acetone, trans-1-phenyl-1-butene and/or 1-phenyl-2-butene.

Ammonia compounds can be selected from the group consisting of ammonia, ammonium hydroxide, ammonium salts, NH₂OH, H₂N-NH₂, and/or an amine having the formula R-NH₂, wherein R is an aliphatic group. R can be for example methyl, ethyl, n-propyl or iso-propyl. For example ammonium sulfate is a suitable ammonia compound.

In one embodiment the hydroamination reaction is a regioselective hydroamination providing the alpha-isomer. Preferably the hydroamination reaction is a stereo-selective reaction preferably providing the S-enantiomer. In one embodiment the method according to the present invention provides the amination product with an enantioselectivity of at least 90 %, preferably at least 95 %, more preferably at least 99%, calculated on the basis of the whole amount of the amination product (e.e). For example the enantioselectivity can be measured using HPLC.

In one embodiment hydroamination reaction is performed with isolated or purified (optionally immobilised) protein having phenylalanine ammonia lyase activity, or in the presence of microorganism expressing a protein having phenylalanine ammonia lyase enzyme activity. Useful microorganisms may be for example Escherichia coli and/or engineered variants of E. coli and/or yeast.

Preferably, the hydroamination reaction in step a) is performed at a pH of from 8.5 to 11, more preferably at a pH of from 9 to 10.5.

Preferably, the hydroamination reaction in step a) is performed at a temperature of from 25 to 35 °C, more preferably at a temperature of from 28 to 33°C.

In one embodiment the hydroamination reaction in step a) is performed with the following concentrations:
0.1 to 60 mM substrate, preferably 5 to 20 mM substrate and/or
1 to 9 M, preferably 4 to 7 M, ammonia compound.

In one embodiment the hydroamination is performed in glycerine, toluene, tetrahydrofuran, n-hexane, iso-hexane, n-heptane, dichloromethane, cyclohexane and/or 1,4-dioxane. Preferably the hydroamination reaction is performed in the presence of 1mmol/L to 5 mmol/L, more preferably 2 mmol/L to 3 mmol/L, Mg²⁺⁻Ions.

In a preferred embodiment the hydroamination reaction is performed in biphasic solvent system. In the biphasic solvent system unpolar solvent and a polar solvent are mixed in a ratio of from 3:1 to 0,5:1, more preferably of from 2:5 to 1.5. Unpolar solvents according to the present inventions mean solvents which are preferably not miscible with water. n-hexane, iso-hexane, acetonitrile, n-heptane and/or cyclohexane are useful unpolar solvents for example. Preferred polar solvents are water or a buffer. For example 0,05 to 0,5 M TRIS-sulfate-Buffer, preferably 0,08 to 0,2M, having a pH of 8 from 10 is a suitable buffer.

According to a further aspect of the invention there is provided the hydroamination product obtainable by the method described above.

### Description of the figures:

Figure 1: Enzymatic deamination of phenylalanine and reverse reaction
Figure 2: Aromatic substrates useful/not useful according to the method of the present invention.
Figure 3: GC-MS reaction: trans-beta-methylstyrene with NH₄OH /*Rhodotorola glutinis (Rt)-*PAL
   Conditions: 0.1 M TRIS-SO₄ (5 mL, pH 10), 10 mM substrate, 4 M NH₄OH, 300 mg whole cells of *Rhodotorola glutinis (Rt)*-PAL, 10 mL *iso*-hexane at 30°C for 4d
Figure 4: GC-MS reaction: trans-beta-methylstyrene with NH₄OH, _{/} *Petroselinum crispum(Pc)*-PAL
   Conditions: 0.1 M TRIS-SO₄ (5 mL, pH 10), 10 mM substrate, 4 M NH₄OH, 300 mg whole cells of *Petroselinum crispum(Pc)*-PAL 10 mL iso-hexane at 30°C for 4d
Figure 5: The enantioselectivity using trans-beta-methylstyrene and ammonia sulfate to produce enantiopure amphetamine (black peak) was proven by comparing the chromatogram with the chromatogram of rac-amphetamine (dotted peaks) using chiral GC-FID

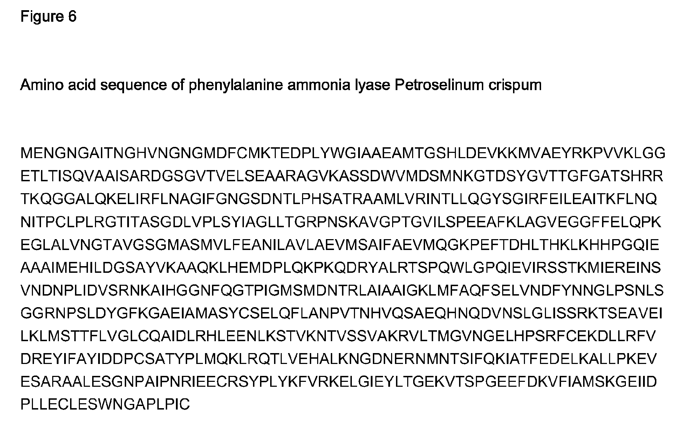

Figure 6: Amino acid sequence of phenylalanine ammonia lyase Petroselinum crispum

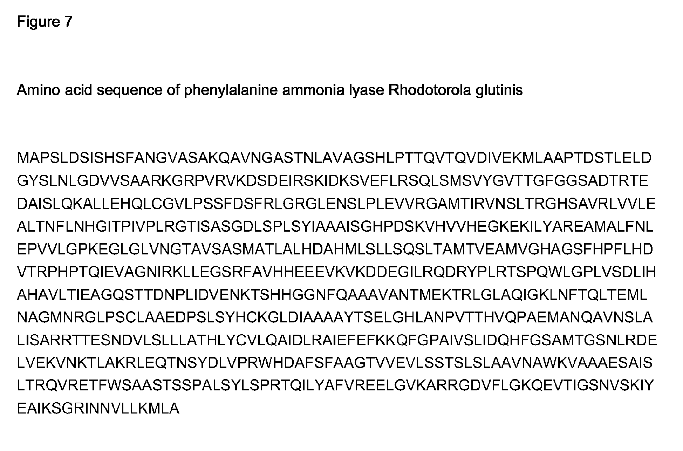

Figure 7: Amino acid sequence of phenylalanine ammonia lyase Rhodotorola glutinis

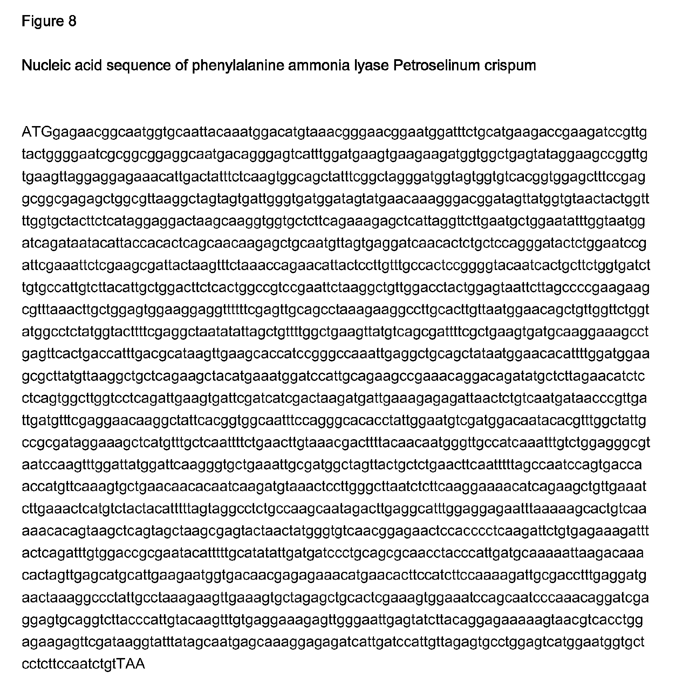

Figure 8: Nucleic acid sequence of phenylalanine ammonia lyase Petroselinum crispum

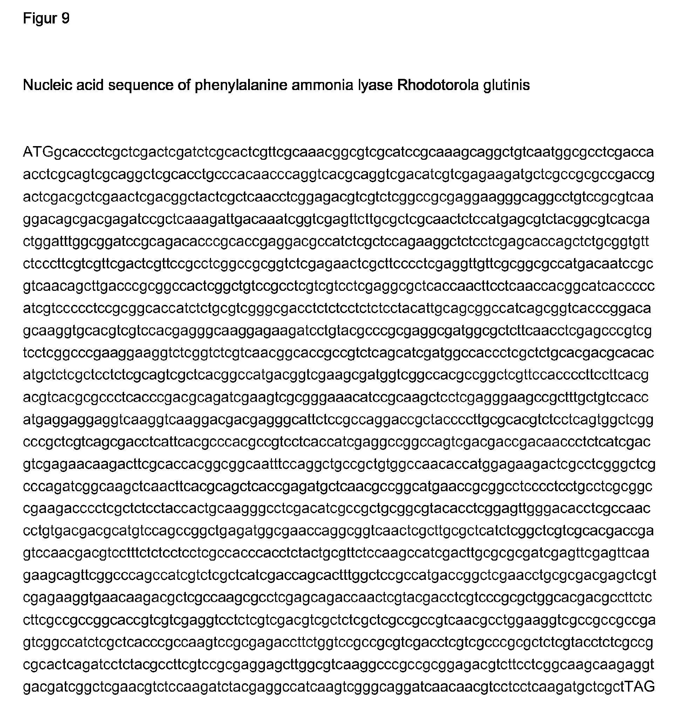

Figure 9: Nucleic acid sequence of phenylalanine ammonia lyase Rhodotorola glutinis

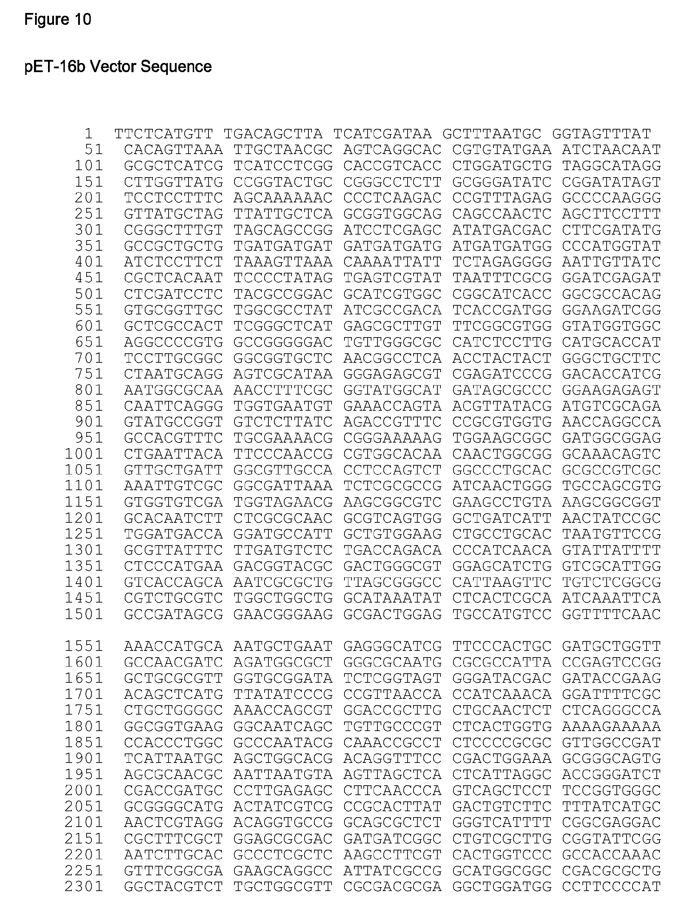

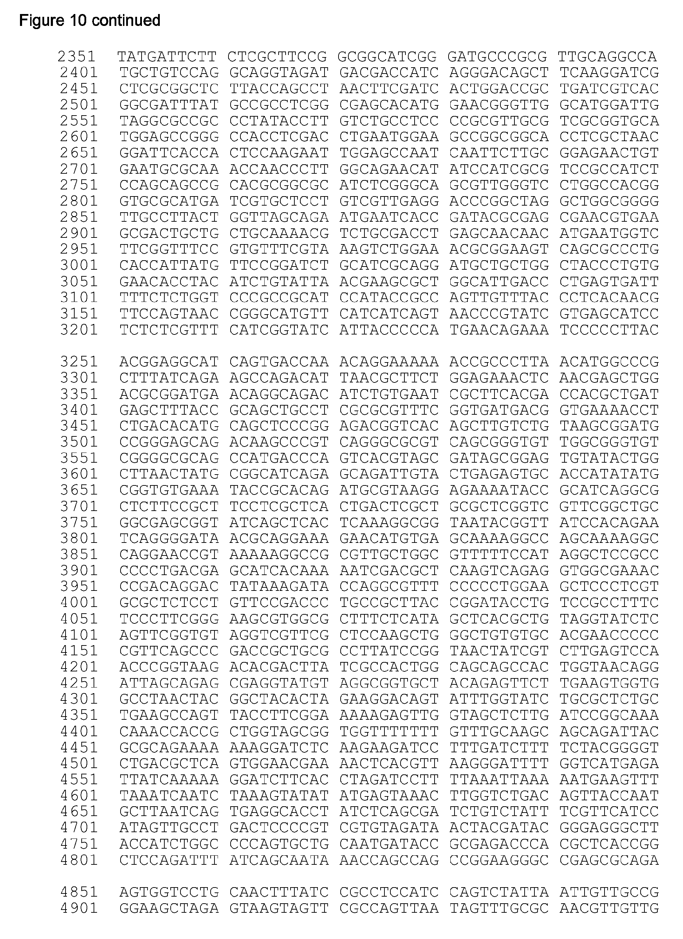

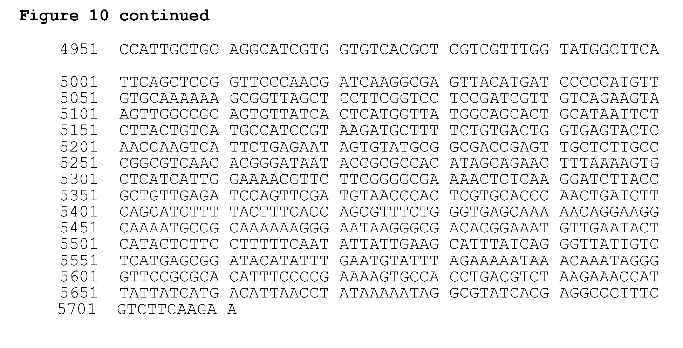

Figure10: Nucleic acid sequence of pET-16b Vector
Figure 11: Vector map of rtPAL-pET 16b

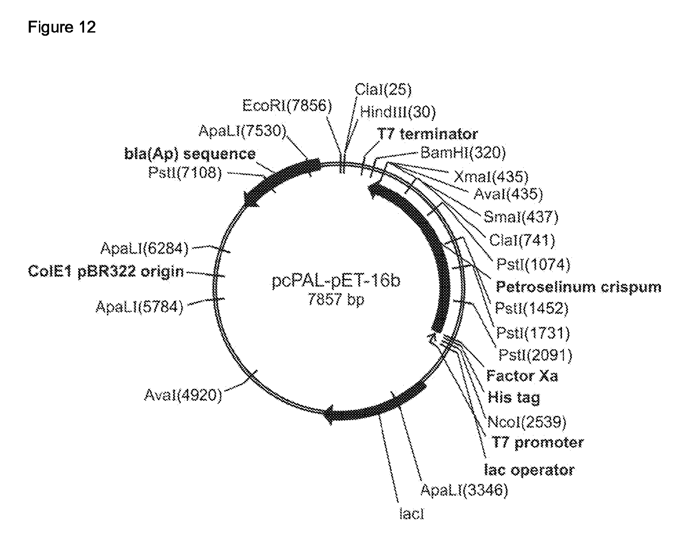

Figure 12: Vector map of pcPAL-pET 16b

### Definitions

A protein having phenylalanine lyase activity catalyses the addition of an amino compound to a double bond in a side chain of a substrate, wherein the amino compound is added to the double bond preferably in the alpha-position. Preferred substrates are defined above.

The method according to the present invention is not limited to the specifically disclosed "proteins with phenylalanine ammonia lyase activity", but also extends to functional equivalents thereof. The term functional equivalents may also include mutated proteins as defined above.

"Functional equivalents" or analogs of the concretely disclosed enzymes are, within the scope of the present invention, various polypeptides thereof, which moreover possess the desired biological function or activity, e.g. enzyme activity.

For example, "functional equivalents" means enzymes, which, in a test used for enzymatic activity, display at least a 1 to 10%, or at least 20%, or at least 50%, or at least 80%, or at least 90% higher or lower activity of an enzyme, as defined herein.

"Functional equivalents", according to the invention, also means in particular mutants, which, in at least one sequence position of the amino acid sequences stated above, have an amino acid that is different from that concretely stated, but nevertheless possess one of the aforementioned biological activities. "Functional equivalents" thus comprise the mutants obtainable by one or more amino acid additions, substitutions, deletions and/or inversions, where the stated changes can occur in any sequence position, provided they lead to a mutant with the profile of properties according to the invention. Functional equivalence is in particular also provided if the reactivity patterns coincide qualitatively between the mutant and the unchanged polypeptide, i.e. if for example the same substrates are converted at a different rate. Examples of suitable amino acid substitutions are shown in the following table:

| Original residue | Examples of substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

| | |
|---|---|
| "Functional equivalents" in the above sense are also "precursors" of the polypeptides described, as well as "functional derivatives" and "salts" of the polypeptides. "Precursors" are in that case natural or synthetic precursors of the polypeptides with or without the desired biological activity. | |

The expression "salts" means salts of carboxyl groups as well as salts of acid addition of amino groups of the protein molecules according to the invention. Salts of carboxyl groups can be produced in a known way and comprise inorganic salts, for example sodium, calcium, ammonium, iron and zinc salts, and salts with organic bases, for example amines, such as triethanolamine, arginine, lysine, piperidine and the like. Salts of acid addition, for example salts with inorganic acids, such as hydrochloric acid and/or sulfuric acid and/or salts with organic acids, such as acetic acid and/or oxalic acid, are also covered by the invention.

"Functional derivatives" of polypeptides according to the invention can also be produced on functional amino acid side groups or at their N-terminal or C-terminal end using known techniques. Such derivatives comprise for example aliphatic esters of carboxylic acid groups, amides of carboxylic acid groups, obtainable by reaction with ammonia or with a primary or secondary amine; N-acyl derivatives of free amino groups, produced by reaction with acyl groups; or O-acyl derivatives of free hydroxy groups, produced by reaction with acyl groups.

"Functional equivalents" naturally also comprise polypeptides that can be obtained from other organisms, as well as naturally occurring variants. For example, areas of homologous sequence regions can be established by sequence comparison, and equivalent enzymes can be determined on the basis of the concrete parameters of the invention.

"Functional equivalents" also comprise fragments, preferably individual domains or sequence motifs, of the polypeptides according to the invention, which for example display the desired biological function.

"Functional equivalents" are, moreover, fusion proteins, which have one of the polypeptide sequences stated above or functional equivalents derived there from and at least one further, functionally different, heterologous sequence in functional N-terminal or C-terminal association (i.e. without substantial mutual functional impairment of the fusion protein parts). Non-limiting examples of these heterologous sequences are e.g. signal peptides, histidine anchors or enzymes.

"Functional equivalents" that are also included according to the invention are homologues of the concretely disclosed proteins. These possess percent identity values as stated above. Said values refer to the identity with the concretely disclosed amino acid sequences, and may be calculated according to the algorithm of Pearson and Lipman, Proc. Natl. Acad, Sci. (USA) 85(8), 1988, 2444-2448.

The % identity values may also be calculated from BLAST alignments, algorithm blastp (protein-protein BLAST) or by applying the Clustal setting as given below.

A percentage identity of a polypeptide according to the invention means in particular the percentage identity of the amino acid residues relative to the total length of one of the amino acid sequences concretely described herein.

In the case of a possible protein glycosylation, "functional equivalents" according to the invention comprise proteins of the type designated above in deglycosylated or glycosylated form as well as modified forms that can be obtained by altering the glycosylation pattern. Such functional equivalents or homologues of the proteins or polypeptides according to the invention can be produced by mutagenesis, e.g. by point mutation, lengthening or shortening of the protein.

Such functional equivalents or homologues of the proteins according to the invention can be identified by screening combinatorial databases of mutants, for example shortening mutants. For example, a variegated database of protein variants can be produced by combinatorial mutagenesis at the nucleic acid level, e.g. by enzymatic ligation of a mixture of synthetic oligonucleotides. There are a many methods that can be used for the production of databases of potential homologues from a degenerated oligonucleotide sequence. Chemical synthesis of a degenerated gene sequence can be carried out in an automatic DNA synthesiser, and the synthetic gene can then be ligated in a suitable expression vector. The use of a degenerated genome makes it possible to supply all sequences in a mixture, which code for the desired set of potential protein sequences. Methods of synthesis of degenerated oligonucleotides are known to a person skilled in the art (e.g. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

In the prior art, several techniques are known for the screening of gene products of combinatorial databases, which were produced by point mutations or shortening, and for the screening of cDNA libraries for gene products with a selected property. These techniques can be adapted for the rapid screening of the gene banks that were produced by combinatorial mutagenesis of homologues according to the invention. The techniques most frequently used for the screening of large gene banks, which are based on a high-throughput analysis, comprise cloning of the gene bank in expression vectors that can be replicated, transformation of the suitable cells with the resultant vector database and expression of the combinatorial genes in conditions in which detection of the desired activity facilitates isolation of the vector that codes for the gene whose product was detected. Recursive Ensemble Mutagenesis (REM), a technique that increases the frequency of functional mutants in the databases, can be used in combination with the screening tests, in order to identify homologues (Arkin and Yourvan (1992) PNAS 89:7811-7815; Delgrave et al. (1993) Protein Engineering 6(3):327-331).

The invention also relates to a method wherein the protein having phenylalanine activity is coded by nucleic acid sequences as defined herein and with a certain degree of "identity" to the sequences specifically disclosed herein. "Identity" between two nucleic acids means identity of the nucleotides, in each case over the entire length of the nucleic acid.

For example the identity may be calculated by means of the Vector NTI Suite 7.1 program of the company Informax (USA) employing the Clustal Method (Higgins DG, Sharp PM. Fast and sensitive multiple sequence alignments on a microcomputer. Comput Appl. Biosci. 1989 Apr; 5(2):151-1) with the following settings:
Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| | |
|---|---|
| FAST algorithm | on |
| K-tuple size | 1 |
| Gap penalty | 3 |
| Window size | 5 |
| Number of best diagonals | 5 |

Alternatively the identity may be determined according to Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, the web page: http:/fwww.ebi.ac.uk/Tools/clustalw/index.html# and the following settings

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

All the nucleic acid sequences mentioned herein (single-stranded and double-stranded DNA and RNA sequences, for example cDNA and mRNA) can be produced in a known way by chemical synthesis from the nucleotide building blocks, e.g. by fragment condensation of individual overlapping, complementary nucleic acid building blocks of the double helix. Chemical synthesis of oligonucleotides can, for example, be performed in a known way, by the phosphoamidite method (Voet, Voet, 2nd edition, Wiley Press, New York, pages 896-897). The accumulation of synthetic oligonucleotides and filling of gaps by means of the Klenow fragment of DNA polymerase and ligation reactions as well as general cloning techniques are described in Sambrook et al. (1989), see below.

The invention also relates a method using nucleic acid sequences (singly-stranded and double stranded DNA and RNA sequences, e.g. cDNA and mRNA), coding for one of the above polypeptides and their functional equivalents, which can be obtained for example using artificial nucleotide analogs. In particular, the nucleic acid sequences may be adapted to the host used for expression.

The nucleic acid molecules defined according to the invention can in addition contain non-translated sequences from the 3' and/or 5' end of the coding genetic region.

A nucleic acid molecule defined according to the invention can be isolated by means of standard techniques of molecular biology and the sequence information supplied according to the invention. For example, cDNA can be isolated from a suitable cDNA library, using one of the concretely disclosed complete sequences or a segment thereof as hybridisation probe and standard hybridisation techniques (as described for example in Sambrook, J., Fritsch, E.F. and Maniatis, T. Molecular Cloning: A Laboratory Manual. 2nd edition, Cold Spring Harbor Laboratory, Cold Spring Harbor Laboratory Press, Cold Spring Harbor, NY, 1989). In addition, a nucleic acid molecule comprising one of the disclosed sequences or a segment thereof, can be isolated by the polymerase chain reaction, using the oligonucleotide primers that were constructed on the basis of this sequence. The nucleic acid amplified in this way can be cloned in a suitable vector and can be characterised by DNA sequencing. The oligonucleotides according to the invention can also be produced by standard methods of synthesis, e.g. using an automatic DNA synthesiser.

Nucleic acid sequences useful for the method according to the invention or derivatives thereof, homologues or parts of these sequences, can for example be isolated by usual hybridisation techniques or the PCR technique from other bacteria, e.g. via genomic or cDNA libraries. These DNA sequences hybridise in standard conditions with the sequences according to the invention.

"Hybridise" means the ability of a polynucleotide or oligonucleotide to bind to an almost complementary sequence in standard conditions, whereas nonspecific binding does not occur between non-complementary partners in these conditions. For this, the sequences can be 90-100% complementary. The property of complementary sequences of being able to bind specifically to one another is utilised for example in Northern Blotting or Southern Blotting or in primer binding in PCR or RT-PCR.

Short oligonucleotides of the conserved regions are used advantageously for hybridisation. However, it is also possible to use longer fragments of the nucleic acids according to the invention or the complete sequences for the hybridisation. These standard conditions vary depending on the nucleic acid used (oligonucleotide, longer fragment or complete sequence) or depending on which type of nucleic acid - DNA or RNA - is used for hybridisation. For example, the melting temperatures for DNA:DNA hybrids are approx. 10°C lower than those of DNA:RNA hybrids of the same length.

For example, depending on the particular nucleic acid, standard conditions mean temperatures between 42 and 58°C in an aqueous buffer solution with a concentration between 0.1 to 5 x SSC (1 X SSC = 0.15 M NaCl, 15 mM sodium citrate, pH 7.2) or additionally in the presence of 50% formamide, for example 42°C in 5 x SSC, 50% formamide. Advantageously, the hybridisation conditions for DNA:DNA hybrids are 0.1 x SSC and temperatures between about 20°C to 45°C, preferably between about 30°C to 45°C. For DNA:RNA hybrids the hybridisation conditions are advantageously 0.1 x SSC and temperatures between about 30°C to 55°C, preferably between about 45°C to 55°C. These stated temperatures for hybridisation are examples of calculated melting temperature values for a nucleic acid with a length of approx. 100 nucleotides and a G + C content of 50% in the absence of formamide. The experimental conditions for DNA hybridisation are described in relevant genetics textbooks, for example Sambrook et al., 1989, and can be calculated using formulae that are known by a person skilled in the art, for example depending on the length of the nucleic acids, the type of hybrids or the G + C content. A person skilled in the art can obtain further information on hybridisation from the following textbooks: Ausubel et al. (eds), 1985, Current Protocols in Molecular Biology, John Wiley & Sons, New York; Hames and Higgins (eds), 1985, Nucleic Acids Hybridisation: A Practical Approach, IRL Press at Oxford University Press, Oxford; Brown (ed), 1991, Essential Molecular Biology: A Practical Approach, IRL Press at Oxford University Press, Oxford.

"Hybridisation" can in particular be carried out under stringent conditions. Such hybridisation conditions are for example described in Sambrook, J., Fritsch, E.F., Maniatis, T., in: Molecular Cloning (A Laboratory Manual), 2nd edition, Cold Spring Harbor Laboratory Press, 1989, pages 9.31-9.57 or in Current Protocols in Molecular Biology, John Wiley & Sons, N.Y. (1989), 6.3.1-6.3.6.

"Stringent" hybridisation conditions mean in particular: Incubation at 42°C overnight in a solution consisting of 50% formamide, 5 x SSC (750 mM NaCl, 75 mM tri-sodium citrate), 50 mM sodium phosphate (pH 7.6), 5x Denhardt Solution, 10% dextran sulfate and 20 g/ml denatured, sheared salmon sperm DNA, followed by washing of the filters with 0.1x SSC at 65°C.

The invention also relates to a method using derivatives of the concretely disclosed or derivable nucleic acid sequences.

Thus, further nucleic acid sequences defined according to the invention can be derived from the sequences specifically disclosed herein and can differ from it by addition, substitution, insertion or deletion of individual or several nucleotides, and furthermore code for polypeptides with the desired profile of properties.

The invention also encompasses methods using nucleic acid sequences that comprise so-called silent mutations or have been altered, in comparison with a concretely stated sequence, according to the codon usage of a special original or host organism, as well as naturally occurring variants, e.g. splicing variants or allelic variants, thereof. In particular, the invention also encompasses coding sequence identified in a organism which codon usage has been optimised for the respective host plants or plant compartments such as tissues or plastids codon usage and/or where cryptic splice sites, polyadenylation signals and/or secondary structures of the transcript have been removed or avoided.

It also relates methods based on sequences that can be obtained by conservative nucleotide substitutions (i.e. the amino acid in question is replaced by an amino acid of the same charge, size, polarity and/or solubility).

The invention also relates to methods based on the molecules derived from the concretely disclosed nucleic acids by sequence polymorphisms. These genetic polymorphisms can exist between individuals within a population owing to natural variation. These natural variations usually produce a variance of 1 to 5% in the nucleotide sequence of a gene.

Derivatives of nucleic acid sequences defined according to the invention mean for example allelic variants, having at least 60% homology at the level of the derived amino acid, preferably at least 80% homology, quite especially preferably at least 90% homology over the entire sequence range (regarding homology at the amino acid level, reference should be made to the details given above for the polypeptides or proteins). Advantageously, the homologies can be higher over partial regions of the sequences.

Furthermore, derivatives are also to be understood to be homologues of the nucleic acid sequences according to the invention, for example animal, plant, fungal or bacterial homologues, shortened sequences, single-stranded DNA or RNA of the coding and noncoding DNA sequence. For example, homologues have, at the DNA level, a homology of at least 40%, preferably of at least 60%, especially preferably of at least 70%, quite especially preferably of at least 80% over the entire DNA region given in a sequence specifically disclosed herein.

Moreover, derivatives are to be understood to be, for example, fusions with promoters. The promoters that are added to the stated nucleotide sequences can be modified by at least one nucleotide exchange, at least one insertion, inversion and/or deletion, though without impairing the functionality or efficacy of the promoters. Moreover, the efficacy of the promoters can be increased by altering their sequence or can be exchanged completely with more effective promoters even of organisms of a different genus.

The phenylalanine ammonia lyase may be expressed by an expression construct.

Expression constructs useful for the method according to the present invention contain a nucleic acid sequence coding for a polypeptide or fusion protein of phenyl alanin ammonia lyase under the genetic control of regulatory nucleic acid sequences. Preferably the expression construct is part of a vector .

Preferably such constructs according to the invention comprise a promoter 5'-upstream from the respective coding sequence, and a terminator sequence 3'-downstream, and optionally further usual regulatory elements, in each case functionally associated with the coding sequence.

A "promoter", a "nucleic acid with promoter activity" or a "promoter sequence" mean, according to the invention, a nucleic acid which, functionally associated with a nucleic acid that is to be transcribed, regulates the transcription of this nucleic acid.

"Functional" association means, in this context, for example the sequential arrangement of one of the nucleic acids with promoter activity and of a nucleic acid sequence that is to be transcribed and optionally further regulatory elements, for example nucleic acid sequences that enable the transcription of nucleic acids, and for example a terminator, in such a way that each of the regulatory elements can fulfill its function in the transcription of the nucleic acid sequence. This does not necessarily require a direct association in the chemical sense. Genetic control sequences, such as enhancer sequences, can also exert their function on the target sequence from more remote positions or even from other DNA molecules. Arrangements are preferred in which the nucleic acid sequence that is to be transcribed is positioned behind (i.e. at the 3' end) the promoter sequence, so that the two sequences are bound covalently to one another. The distance between the promoter sequence and the nucleic acid sequence that is to be expressed transgenically can be less than 200 bp (base pairs), or less than 100 bp or less than 50 bp.

Apart from promoters and terminators, examples of other regulatory elements that may be mentioned are targeting sequences, enhancers, polyadenylation signals, selectable markers, amplification signals, replication origins and the like. Suitable regulatory sequences are described for example in Goeddel, Gene Expression Technology: Methods in Enzymology 185, Academic Press, San Diego, CA (1990).

Examples of suitable regulatory sequences are contained in promoters such as cos-, tac-, trp-, tet-, trp-tet-, lpp-, lac-, lpp-lac-, lacl^{q-} , T7-, T5-, T3-, gal-, trc-, ara-, rhaP (rhaP_{BAD})SP6-, lambda-P_{R}- or in the lambda-P_{L}-promoter, which find application advantageously in Gram-negative bacteria. Other advantageous regulatory sequences are contained for example in the Gram-positive promoters ace, amy and SPO2, in the yeast or fungal promoters ADC1, MFalpha, AC, P-60, CYC1, GAPDH, TEF, rp28, ADH. Artificial promoters can also be used for regulation.

For expression, the nucleic acid construct is inserted in a host organism advantageously in a vector, for example a plasmid or a phage, which permits optimum expression of the genes in the host. In addition to plasmids and phages, vectors are also to be understood as meaning all other vectors known to a person skilled in the art, e.g. viruses, such as SV40, CMV, baculovirus and adenovirus, transposons, IS elements, phasmids, cosmids, linear or circular DNA. These vectors can be replicated autonomously in the host organism or can be replicated chromosomally.

Suitable plasmids are, for example in *E. coli,* pLG338, pACYC184, pBR322, pUC18, pET16b, pUC19, pKC30, pRep4, pHS1, pKK223-3, pDHE19.2, pHS2, pPLc236, pMBL24, pLG200, pUR290, pIN-III¹¹³-B1, λgt11 or pBdCl; in nocardioform actinomycetes pJAM2; in *Streptomyces* plJ101, plJ364, plJ702 or plJ361; in bacillus pUB110, pC194 or pBD214; in *Corynebacterium* pSA77 or pAJ667; in fungi pALS1, pIL2 or pBB116; in yeasts 2alphaM, pAG-1, YEp6, YEp13 or pEMBLYe23 or in plants pLGV23, pGHlac⁺, pBIN19, pAK2004 or pDH51. The aforementioned plasmids represent a small selection of the possible plasmids. Other plasmids are well known to a person skilled in the art and will be found for example in the book Cloning Vectors (Eds. Pouwels P.H. et al. Elsevier, Amsterdam-New York-Oxford, 1985, ISBN 0 444 904018).

The production of a recombinant expression construct useful for the production of the phenylalanine ammonia lyase is based on fusion of a suitable promoter with a suitable coding nucleotide sequence and a terminator signal or polyadenylation signal. Common recombination and cloning techniques are used for this, as described for example in T. Maniatis, E.F. Fritsch and J. Sambrook, Molecular Cloning: A Laboratory Manual, Cold Spring Harbor Laboratory, Cold Spring Harbor, NY (1989) as well as in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

The recombinant expression construct is inserted advantageously in a host-specific vector for expression in a suitable host organism, to permit optimum expression of the genes in the host. Vectors are well known to a person skilled in the art and will be found for example in "Cloning Vectors" (Pouwels P.H. et al., Publ. Elsevier, Amsterdam-New York-Oxford, 1985). By means of the vectors defined above, recombinant microorganisms can be produced, which have been transformed for example with at least one vector according to the invention and can be used for production of phenylalanine ammonia lyase. Advantageously, the recombinant constructs described above, are inserted in a suitable host system and expressed. Preferably, common cloning and transfection methods that are familiar to a person skilled in the art are used, for example co-precipitation, protoplast fusion, electroporation, retroviral transfection and the like, in order to secure expression of the stated nucleic acids in the respective expression system. Suitable systems are described for example in Current Protocols in Molecular Biology, F. Ausubel et al., Publ. Wiley Interscience, New York 1997, or Sambrook et al. Molecular Cloning: A Laboratory Manual. 2nd edition, Cold Spring Harbor Laboratory, Cold Spring Harbor Laboratory Press, Cold Spring Harbor, NY, 1989.

In principle, all prokaryotic organisms can be considered as recombinant host organisms for the nucleic acid according to the invention or the nucleic acid construct. Bacteria are used advantageously as host organisms. Preferably they are selected from native or recombinant bacteria having the ability to produce inclusion bodies of the PHA-, TAG- or WE-type, as in particular the TAG-producing nocardioform actinomycetes, in particular of the genus *Rhodococcus, Mycobacterium, Nocardia, Gordonia, Skermania* and *Tsukamurella*; as well as TAG-producing Streptomycetes; WE-producing genera *Acinetobacter* and *Alcanivorax;* as well as recombinant strains of the genus *Escherichia,* especially *E. coli, Corynebacterium,* especially *C. glutamicum* und *Bacillus,* especially *B*. *subtilis.*

The host organism or host organisms useful according to the invention then preferably contain at least one of the nucleic acid sequences, nucleic acid constructs or vectors described in this invention, which code for an enzyme activity according to the above definition.

The organisms used in the method according to the invention are grown or bred in a manner familiar to a person skilled in the art, depending on the host organism. As a rule, microorganisms are grown in a liquid medium, which contains a source of carbon, generally in the form of sugars, a source of nitrogen generally in the form of organic sources of nitrogen such as yeast extract or salts such as ammonium sulfate, trace elements such as iron, manganese and magnesium salts and optionally vitamins, at temperatures between 0°C and 100°C, preferably between 10°C to 60°C with oxygen aeration. The pH of the liquid nutrient medium can be maintained at a fixed value, i.e. regulated or not regulated during growing. Growing can be carried out batchwise, semi-batchwise or continuously. Nutrients can be supplied at the start of fermentation or can be supplied subsequently, either semi-continuously or continuously.

The invention also relates to methods for production of proteins according to the invention by cultivating a microorganism which expresses said protein, and isolating the desired product from the culture.

The microorganisms as used according to the invention can be cultivated continuously or discontinuously in the batch process or in the fed batch or repeated fed batch process. A review of known methods of cultivation will be found in the textbook by Chmiel (Bioprocesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium that is to be used must satisfy the requirements of the particular strains in an appropriate manner. Descriptions of culture media for various microorganisms are given in the handbook "Manual of Methods for General Bacteriology" of the American Society for Bacteriology (Washington D. C., USA, 1981).

These media that can be used according to the invention generally comprise one or more sources of carbon, sources of nitrogen, inorganic salts, vitamins and/or trace elements.

Preferred sources of carbon are sugars, such as mono-, di- or polysaccharides. Very good sources of carbon are for example glucose, fructose, mannose, galactose, ribose, sorbose, ribulose, lactose, maltose, sucrose, raffinose, starch or cellulose. Sugars can also be added to the media via complex compounds, such as molasses, or other by-products from sugar refining. It may also be advantageous to add mixtures of various sources of carbon. Other possible sources of carbon are oils and fats such as soybean oil, sunflower oil, peanut oil and coconut oil, fatty acids such as palmitic acid, stearic acid or linoleic acid, alcohols such as glycerol, methanol or ethanol and organic acids such as acetic acid or lactic acid. Sources of nitrogen are usually organic or inorganic nitrogen compounds or materials containing these compounds. Examples of sources of nitrogen include ammonia gas or ammonium salts, such as ammonium sulfate, ammonium chloride, ammonium phosphate, ammonium carbonate or ammonium nitrate, nitrates, urea, amino acids or complex sources of nitrogen, such as corn-steep liquor, soybean flour, soybean protein, yeast extract, meat extract and others. The sources of nitrogen can be used separately or as a mixture. Inorganic salt compounds that may be present in the media comprise the chloride, phosphate or sulfate salts of calcium, magnesium, sodium, cobalt, molybdenum, potassium, manganese, zinc, copper and iron.

Inorganic sulfur-containing compounds, for example sulfates, sulfites, dithionites, tetra-thionates, thiosulfates, sulfides, but also organic sulfur compounds, such as mercaptans and thiols, can be used as sources of sulfur.

Phosphoric acid, potassium dihydrogenphosphate or dipotassium hydrogenphosphate or the corresponding sodium-containing salts can be used as sources of phosphorus. Chelating agents can be added to the medium, in order to keep the metal ions in solution. Especially suitable chelating agents comprise dihydroxyphenols, such as catechol or proto-catechuate, or organic acids, such as citric acid.

The fermentation media used according to the invention may also contain other growth factors, such as vitamins or growth promoters, which include for example biotin, riboflavin, thiamine, folic acid, nicotinic acid, pantothenate and pyridoxine. Growth factors and salts often come from complex components of the media, such as yeast extract, molasses, corn-steep liquor and the like. In addition, suitable precursors can be added to the culture medium. The precise composition of the compounds in the medium is strongly dependent on the particular experiment and must be decided individually for each specific case. Information on media optimisation can be found in the textbook "Applied Microbiol. Physiology, A Practical Approach" (Publ. P.M. Rhodes, P.F. Stanbury, IRL Press (1997) p. 53-73, ISBN 0 19 963577 3). Growing media can also be obtained from commercial suppliers, such as Standard 1 (Merck) or BHI (Brain heart infusion, DIFCO) etc.

All components of the medium are sterilised, either by heating (20 min at 1.5 bar and 121°C) or by sterile filtration. The components can be sterilised either together, or if necessary separately. All the components of the medium can be present at the start of growing, or optionally can be added continuously or by batch feed.

The temperature of the culture is normally between 15°C and 45°C, preferably 25°C to 40°C and can be kept constant or can be varied during the experiment. The pH value of the medium should be in the range from 5 to 8.5, preferably around 7.0. The pH value for growing can be controlled during growing by adding basic compounds such as sodium hydroxide, potassium hydroxide, ammonia or ammonia water or acid compounds such as phosphoric acid or sulfuric acid. Antifoaming agents, e.g. fatty acid polyglycol esters, can be used for controlling foaming. To maintain the stability of plasmids, suitable substances with selective action, e.g. antibiotics, can be added to the medium. Oxygen or oxygen-containing gas mixtures, e.g. the ambient air, are fed into the culture in order to maintain aerobic conditions. The temperature of the culture is normally from 20°C to 45°C. Culture is continued until a maximum of the desired product has formed. This is normally achieved within 10 hours to 160 hours.

The cells can be disrupted optionally by high-frequency ultrasound, by high pressure, e.g. in a French pressure cell, by osmolysis, by the action of detergents, lytic enzymes or organic solvents, by means of homogenisers or by a combination of several of the methods listed.

The following examples only serve to illustrate the invention. The numerous possible variations that are obvious to a person skilled in the art also fall within the scope of the invention.

### Examples

### 1) Instruments and material

The compound *trans*-β-methylstyrene, ammonium hydroxide solution (CAS-#-1336-21-6, 25 % solution), methylamine (70% solution in H₂O, CAS-#74-89-5), ethylamine (70% solution in H₂O), isopropylamine (99%), hydrazine (35% solution in H₂O) and hydroxylamine (50% solution in H₂O) were purchased from Sigma-Aldrich. Ammonium sulfate (CAS-#7783-30-3) was purchased from Fluka. Nde I and BamH I restriction enzymes were purchased from Roche and T4 ligase from New England Biolabs. The digest and ligation were done by Geneart.

GC-MS analysis was performed on an Agilent HP 6890 Series GC system equipped with an Agilent 5973 mass selective detector and a GL Focus autosampler from ATAS.

### 2) Construction of PAL expression vector

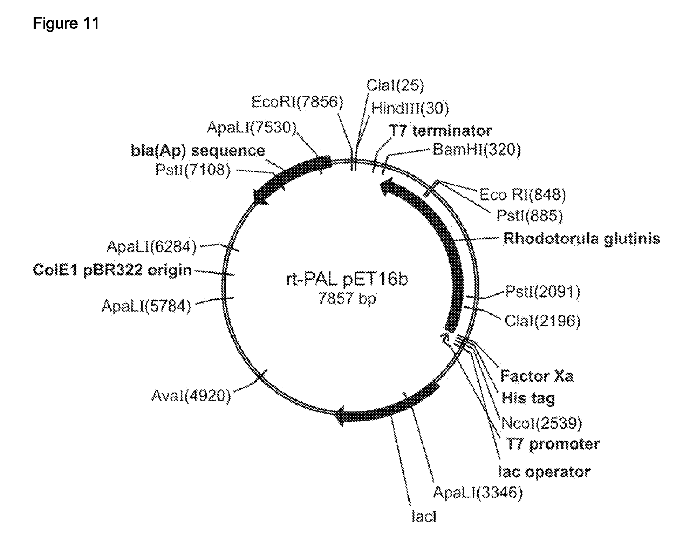

Phenylalanine ammonia lyase Petroselinum crispum and /or Rhodoturula glutinis with optimised codon usage were synthezised by Geneart AG (Regensburg, Germany) and cloned into the plasmid pET-16b from Novagen using Nde I and BamH I restriction sites. The ligation product was transformed into E.coli TOP10 E. coli cells and transformants were plated out on LB-agar plates with ampicillin (cf. vector maps Figure 11).

### 3) Expression of phenylalanine ammonia lyase

The PAL enzymes were produced in *E. coli* BL21 (DE3) following the producer's protocol (Invitrogen). One shot of BL21 (DE3) competent cells (50 µL of cells) was thawed on ice and mixed directly with 1 µL of plasmid DNA. The mixture was stored on ice for 30 min, then incubated for 30 sec at 42°C and returned to ice for 2 min. 250 µL of SOC medium (at room temperature) was added. The vials were left shaking at 37°C for 1 h at 250 rpm. 20-50 µL of each transformation was spread into LB/amp plates and incubated overnight at 37°C. Fresh BL21(DE3) cells that contained the appropriate expression plasmid were collected from a LB/amp plate using a sterile loop and used to inoculate LB medium (10 mL) containing ampicillin (100 µg mL⁻¹, LB/amp medium). Pre-cultures were grown at 37°C to an OD₆₀₀ of 0.5-0.6 with vigorous shaking. The pre-culture (700 µL) was used to inoculate LB/amp medium (700 mL) and then induced with isopropyl-β-D-thiogalactopyranosid (1 mM IPTG). Incubation was continued for 24 h at 26°C. Cells were harvested by centrifugation at 8000 rpm for 15 min, washed with 100 mM potassium phosphate buffer, pH 8.0. Cells were disrupted by sonication for 12 cycles (20 sec on and 20 sec off) in potassium phosphate buffer (100 mM, pH 8, 300 mM KCI, 0 mM imidazole) using phenylmethylsulfonylfluorid (PMSF) and lysozyme, after which unbroken cells and debris were removed by centrifugation (18000 rpm, 10 min). All steps were performed at 4°C. PAL was purified by Ni-based immobilised metal affinity chromatography by using an AKTA system (Amersham). After washing with 0mM and 200 mM imidazole, the bound protein was eluted with 350 mM imidazole. Fractions that contained PAL were desalted and concentrated in storage buffer (100 mM potassium phosphate buffer, pH 8.0). The purified enzyme was stored at +4°C until further use. The purity of the protein was analysed by SDS-PAGE and the protein concentration was determined according to the bicinchoninic acid method (BCA) using bovine serum albumine (BSA) as a standard (Pierce).

### 4) Activity

PAL activity was determined spetrophotometrically by following the formation of the amination product at 270 nm. Standard conditions for the measurement of PAL activity were 0.1 M TRIS buffer pH 8.8, 3 mM phenylalanine at 30°C. For example the formation of the amination product from trans-β-methylstyrene was measured.

### 5) Reaction

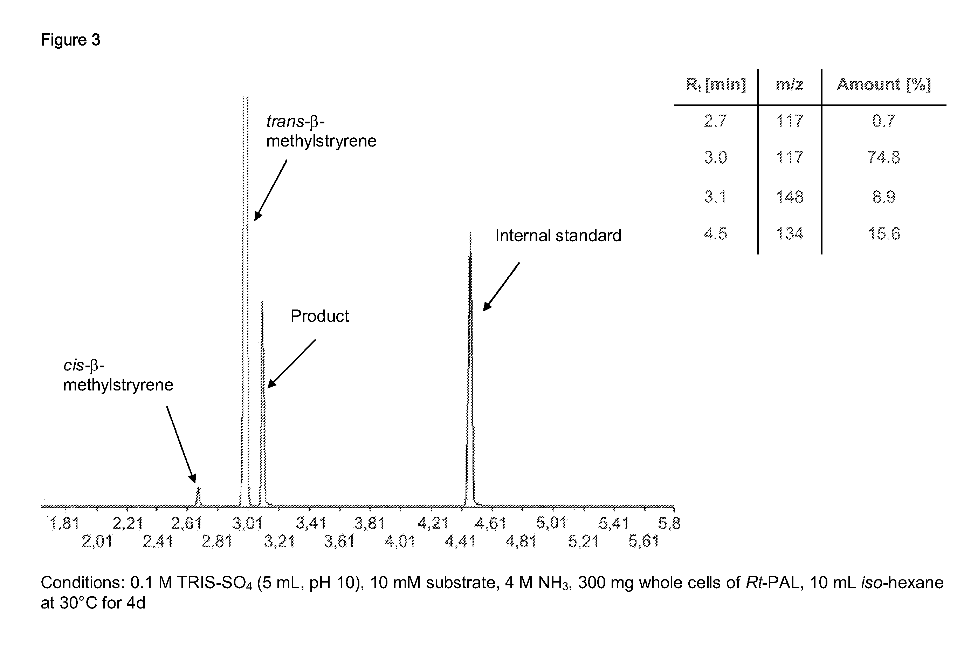

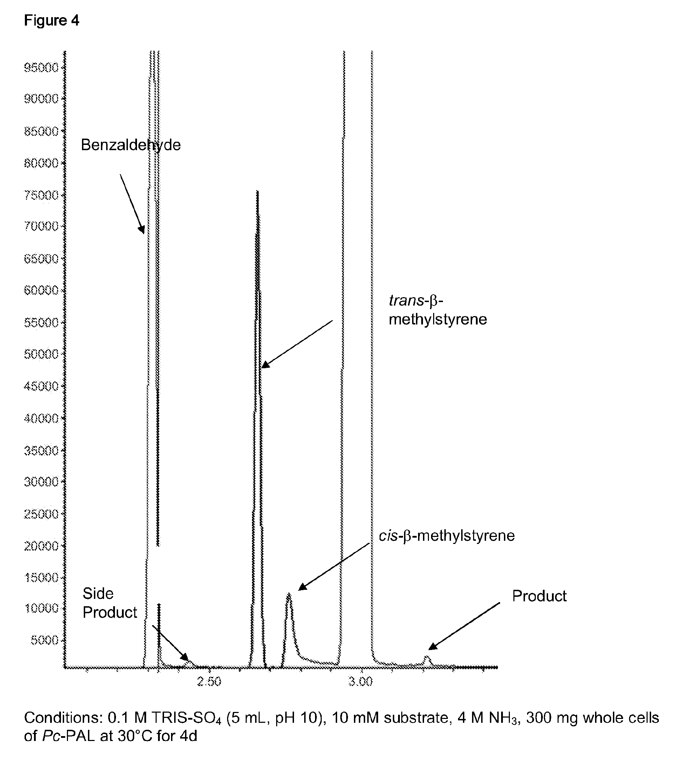

Whole cells of (*Rt*)-PAL or (*Pc*)-PAL (300 mg) were added to trans-β-methylstyrene (10 mM) in a 4 M ammonium hydroxide solution (pH 10, 10 mL total volume). The reaction mixture was incubated for 4 d at 30°C and 250 rpm. The pH was readjusted to 12 with 5 M NaOH and extracted with 4 mL CH₂Cl₂. The organic layer was filtered and evaporated. The precipitate was dissolved in a H₂O/acetonitrile mixture containing 0.1 % formic acid (70/30, 500 µL) and injected to the LC-MS system (Figure 3/4).

### 6) Product identification by LC-MS

LC-MS analysis was performed by using API-ES ionisation in both positive and negative mode. An Exclipse-XDE C18 5µ column (5 µm, 4.6 x 150 mm) was used for analytical separation. The flow rate was 0.5 mL/min and the temperature of the column was maintained at 15°C. Propiophenone (1 µL/mL) was used as internal standard for determining the conversion of reaction. Products obtained from trans-β-methylstyrene biotransformations were eluted with a gradient as follows: start *with 10*/*90 acetonitrilel*H₂O for 12 min, in 10 min from 10/90 to 40/60 and from 40/60 to 10/90 from 22 min to 30 min. The analyses were carried out with UV detection at 220 and 260 nm.

### 7) Reaction using diverse substrates

Whole cells of (*Rt*)-PAL (300 mg) were added to substrate (10 mM) in a 4 M ammonium sulfate solution (pH 10, iso-Hexan, 10 mL total volume). The reaction mixture was incubated for 4 d at 30°C and 250 rpm. The pH was readjusted to 12 with 5 M NaOH and extracted with 4 mL CH₂Cl₂. The organic layer was filtered and evaporated. The precipitate was dissolved in a H₂O/acetonitril mixture containing 0.1% formic acid (70/30, 500 µL) for all others and injected to the LC-MS system.

### 8) Determination of enantiomeric excess of product

For the determination of the enantiomeric excess, the amine formed during biotransformation of trans-beta-methylstyrene was derivatised (Ac₂O, DMAP, EtOAc) to obtain the corresponding acetylated amine in order to achieve enantioseparation on a chiral GC column. An aliquot of the enzyme reaction mixture (0.5 mL) was extracted with 0.5 mL of ethyl acetate and dried over Na₂SO₄. After addition of 0.5 mL of acetic anhydride and cat. DMAP, the Eppendorff vial was shaken at rt for 30 min. The samples were extracted with 0.5 mL of water, the organic phase was dried over Na₂SO₄ and the samples were analysed on chiral GC-FID. GC analyses were carried out on an Agilent 6850 Network gas chromatograph equipped with FID and a Gerstel MPS2L multipurpose autosampler using a Varian CP-Chirasil-DEX CB column (25 m x 0.25 mm x 0.25 µm film) and H₂ as carrier gas. The injector temperature is set 250°C with a flow of 1.7 ml/min H₂ and a temperature program of 150°C - hold 10 min - 15°C/min - 180°C - hold 5 min - 15°C/min - 250°C.

Hydroamination of trans-beta-methylstyrene using different ammonia sources

| | Conversion [%] | e.e. [%] | m/z |
|---|---|---|---|
| Ethylamine | 7 | n.d | 164 |
| Methylamine | 11 | >99a | 150 |
| Hydrazine^{a} | 2 | n.d | 151 |
| Isopropylamine | <1 | n.d. | 178 |
| Ammoniumsulfate | 8 | >99^{a} | 136 |

| | | | |
|---|---|---|---|
| ^{a} by-product formation observed ^{b} conditions: 10 mM substrate, 6 M ammonia sulfate, 300 mg whole cells of Rhodotorula glutinis, 0.1 M TRIS buffer (pH 10) in 10 mL total volume for 3d at 30°C, LC-MS analyses e.e. =S-enantiomer | | | |

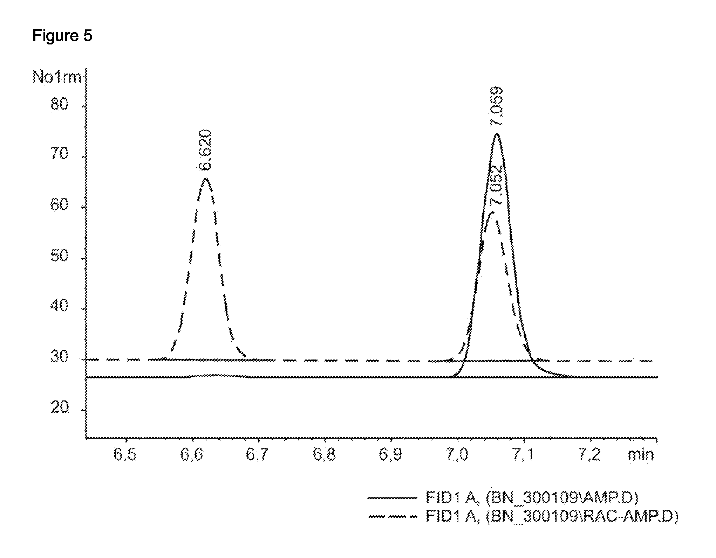

The enantioselectivity using trans-beta-methylstyrene and ammonia sulfate to produce enantiopure amphetamine (black peak) was proven by comparing the chromatogram with the chromatogram of rac-amphetamine (dotted peaks) using chiral GC-FID (Figure 5).

## Claims

1. A method of hydroaminating a double bond in a side chain of an aromatic substrate
which method comprises the following steps:
a) contacting the aromatic substrate in the presence of an ammonia compound with
i) a protein having phenylalanine ammonia lyase activity isolated from Petroselinum crispum and /or Rhodoturula glutinis,
ii) a protein having phenylalanine ammonia lyase activity having a sequence identity of at least 70 % to SEQ-ID No. 1 or to SEQ-ID. No. 2,
iii) a protein coded by a nucleic acid sequence having a sequence identity of at least 70 % to SEQ No. 3 or SEQ No. 4 and/or
iv) functional equivalents of the proteins according to i), ii) and/or iii)
b) and optionally isolating the hydroamination product from the aromatic substrate,
wherein the hydroamination product is not L-tyrosine and/or L-phenylalanine.

2. The method of claim 1, wherein the C-C double bond is not substituted with a carboxyl group and/or a carbon acid ester group.

3. The method of claims 1 or 2,
wherein the aromatic substrate comprises a phenyl group.

4. The method of anyone of the preceding claims, wherein the aromatic substrate is selected from the group consisting of trans-beta-methylstyrene, 4-methoxy cinnamaldehyde, 4-methoxy cinnamonitrile, cis-beta-methylstyrene, cinnamyl chloride, trans-beta-nitrostyrene, styrene, cinnamamide, 4-chloro-alpha-methylstyrene, 4-methoxy stilbene, trans-cinnamaldehyde, 2-phenyl-1-pentene, trans-1-phenyl-1-butene, 2-phenyl-4-penten-2-ol, trans-cinnamonitrile, isoeugenol, 1-phenyl-2-butene, cinnamyl alcohol, coniferyl alcohol, 4-hydroxybenzylideneacetone, trans-anethole, 2-phenyl-2-butene, 2-methyl-1-phenyl-1-butene, 2-methyl-1-phenyl-1-propene, trans-1-phenyl-1-pentene, trans-6-phenyl-2-hexene, trans-2-hexene and trans-2-pentene and mixtures thereof.

5. The method of anyone of the preceding claims, wherein the ammonia compound is selected from the group consisting of ammonia, ammonium hydroxide, ammonium salts, H₂N-NH₂, NH₂OH, NH₂CH₂OH and/or an amine having the formula R-NH_{2,} wherein R is an aliphatic group.

6. The method of anyone of the preceding claims, wherein the hydroamination reaction is a stereo-selective hydroamination providing the S-enantioamer.

7. The method of anyone of the preceding claims, wherein the hydroamination reaction is performed with isolated or purified (optionally immobilised) protein having phenylalanine ammonia lyase activity, or in the presence of microorganism expressing a protein having phenylalanine ammonia lyase enzyme activity.

8. The method according to anyone of the preceding claims, wherein the hydroamination reaction in step a) is performed at a pH of from 8.5 to 11.

9. The method according to anyone of the preceding claims, wherein the hydroamination reaction in step a) is performed at a temperature of from 25 to 35 °C.

10. The method according to anyone of the preceding claims, wherein
the hydroamination reaction in step a) is performed with the following concentrations:
0.1 to 60 mM aromatic substrate and/or
1 to 9 M ammonia compound.

11. The method according to anyone of the preceding claims, wherein
the hydroamination reaction in step a) is performed in the presence of 1mmol/L to 5 mmol/L Mg²⁺⁻Ions.

12. Hydroamination product obtainable by the method according to anyone of the preceding claims.


SEQUENCE LISTING

<110>  BASF SE

<120>  Biocatalyst for Catalytic Hydroamination

<130>  PF 62435

<160>  5

<170>  PatentIn version 3.4

<210>  1
<211>  716
<212>  PRT
<213>  Petroselinum crispum

<400>  1

Met Glu Asn Gly Asn Gly Ala Ile Thr Asn Gly His Val Asn Gly Asn
1               5                   10                  15

Gly Met Asp Phe Cys Met Lys Thr Glu Asp Pro Leu Tyr Trp Gly Ile
            20                  25                  30

Ala Ala Glu Ala Met Thr Gly Ser His Leu Asp Glu Val Lys Lys Met
        35                  40                  45

Val Ala Glu Tyr Arg Lys Pro Val Val Lys Leu Gly Gly Glu Thr Leu
    50                  55                  60

Thr Ile Ser Gln Val Ala Ala Ile Ser Ala Arg Asp Gly Ser Gly Val
65                  70                  75                  80

Thr Val Glu Leu Ser Glu Ala Ala Arg Ala Gly Val Lys Ala Ser Ser
                85                  90                  95

Asp Trp Val Met Asp Ser Met Asn Lys Gly Thr Asp Ser Tyr Gly Val
            100                 105                 110

Thr Thr Gly Phe Gly Ala Thr Ser His Arg Arg Thr Lys Gln Gly Gly
        115                 120                 125

Ala Leu Gln Lys Glu Leu Ile Arg Phe Leu Asn Ala Gly Ile Phe Gly
    130                 135                 140

Asn Gly Ser Asp Asn Thr Leu Pro His Ser Ala Thr Arg Ala Ala Met
145                 150                 155                 160

Leu Val Arg Ile Asn Thr Leu Leu Gln Gly Tyr Ser Gly Ile Arg Phe
                165                 170                 175

Glu Ile Leu Glu Ala Ile Thr Lys Phe Leu Asn Gln Asn Ile Thr Pro
            180                 185                 190

Cys Leu Pro Leu Arg Gly Thr Ile Thr Ala Ser Gly Asp Leu Val Pro
        195                 200                 205

Leu Ser Tyr Ile Ala Gly Leu Leu Thr Gly Arg Pro Asn Ser Lys Ala
    210                 215                 220

Val Gly Pro Thr Gly Val Ile Leu Ser Pro Glu Glu Ala Phe Lys Leu
225                 230                 235                 240

Ala Gly Val Glu Gly Gly Phe Phe Glu Leu Gln Pro Lys Glu Gly Leu
                245                 250                 255

Ala Leu Val Asn Gly Thr Ala Val Gly Ser Gly Met Ala Ser Met Val
            260                 265                 270

Leu Phe Glu Ala Asn Ile Leu Ala Val Leu Ala Glu Val Met Ser Ala
        275                 280                 285

Ile Phe Ala Glu Val Met Gln Gly Lys Pro Glu Phe Thr Asp His Leu
    290                 295                 300

Thr His Lys Leu Lys His His Pro Gly Gln Ile Glu Ala Ala Ala Ile
305                 310                 315                 320

Met Glu His Ile Leu Asp Gly Ser Ala Tyr Val Lys Ala Ala Gln Lys
                325                 330                 335

Leu His Glu Met Asp Pro Leu Gln Lys Pro Lys Gln Asp Arg Tyr Ala
            340                 345                 350

Leu Arg Thr Ser Pro Gln Trp Leu Gly Pro Gln Ile Glu Val Ile Arg
        355                 360                 365

Ser Ser Thr Lys Met Ile Glu Arg Glu Ile Asn Ser Val Asn Asp Asn
    370                 375                 380

Pro Leu Ile Asp Val Ser Arg Asn Lys Ala Ile His Gly Gly Asn Phe
385                 390                 395                 400

Gln Gly Thr Pro Ile Gly Met Ser Met Asp Asn Thr Arg Leu Ala Ile
                405                 410                 415

Ala Ala Ile Gly Lys Leu Met Phe Ala Gln Phe Ser Glu Leu Val Asn
            420                 425                 430

Asp Phe Tyr Asn Asn Gly Leu Pro Ser Asn Leu Ser Gly Gly Arg Asn
        435                 440                 445

Pro Ser Leu Asp Tyr Gly Phe Lys Gly Ala Glu Ile Ala Met Ala Ser
    450                 455                 460

Tyr Cys Ser Glu Leu Gln Phe Leu Ala Asn Pro Val Thr Asn His Val
465                 470                 475                 480

Gln Ser Ala Glu Gln His Asn Gln Asp Val Asn Ser Leu Gly Leu Ile
                485                 490                 495

Ser Ser Arg Lys Thr Ser Glu Ala Val Glu Ile Leu Lys Leu Met Ser
            500                 505                 510

Thr Thr Phe Leu Val Gly Leu Cys Gln Ala Ile Asp Leu Arg His Leu
        515                 520                 525

Glu Glu Asn Leu Lys Ser Thr Val Lys Asn Thr Val Ser Ser Val Ala
    530                 535                 540

Lys Arg Val Leu Thr Met Gly Val Asn Gly Glu Leu His Pro Ser Arg
545                 550                 555                 560

Phe Cys Glu Lys Asp Leu Leu Arg Phe Val Asp Arg Glu Tyr Ile Phe
                565                 570                 575

Ala Tyr Ile Asp Asp Pro Cys Ser Ala Thr Tyr Pro Leu Met Gln Lys
            580                 585                 590

Leu Arg Gln Thr Leu Val Glu His Ala Leu Lys Asn Gly Asp Asn Glu
        595                 600                 605

Arg Asn Met Asn Thr Ser Ile Phe Gln Lys Ile Ala Thr Phe Glu Asp
    610                 615                 620

Glu Leu Lys Ala Leu Leu Pro Lys Glu Val Glu Ser Ala Arg Ala Ala
625                 630                 635                 640

Leu Glu Ser Gly Asn Pro Ala Ile Pro Asn Arg Ile Glu Glu Cys Arg
                645                 650                 655

Ser Tyr Pro Leu Tyr Lys Phe Val Arg Lys Glu Leu Gly Ile Glu Tyr
            660                 665                 670

Leu Thr Gly Glu Lys Val Thr Ser Pro Gly Glu Glu Phe Asp Lys Val
        675                 680                 685

Phe Ile Ala Met Ser Lys Gly Glu Ile Ile Asp Pro Leu Leu Glu Cys
    690                 695                 700

Leu Glu Ser Trp Asn Gly Ala Pro Leu Pro Ile Cys
705                 710                 715

<210>  2
<211>  716
<212>  PRT
<213>  Rhodotorola glutinis

<400>  2

Met Ala Pro Ser Leu Asp Ser Ile Ser His Ser Phe Ala Asn Gly Val
1               5                   10                  15

Ala Ser Ala Lys Gln Ala Val Asn Gly Ala Ser Thr Asn Leu Ala Val
            20                  25                  30

Ala Gly Ser His Leu Pro Thr Thr Gln Val Thr Gln Val Asp Ile Val
        35                  40                  45

Glu Lys Met Leu Ala Ala Pro Thr Asp Ser Thr Leu Glu Leu Asp Gly
    50                  55                  60

Tyr Ser Leu Asn Leu Gly Asp Val Val Ser Ala Ala Arg Lys Gly Arg
65                  70                  75                  80

Pro Val Arg Val Lys Asp Ser Asp Glu Ile Arg Ser Lys Ile Asp Lys
                85                  90                  95

Ser Val Glu Phe Leu Arg Ser Gln Leu Ser Met Ser Val Tyr Gly Val

            100                 105                 110

Thr Thr Gly Phe Gly Gly Ser Ala Asp Thr Arg Thr Glu Asp Ala Ile
        115                 120                 125

Ser Leu Gln Lys Ala Leu Leu Glu His Gln Leu Cys Gly Val Leu Pro
    130                 135                 140

Ser Ser Phe Asp Ser Phe Arg Leu Gly Arg Gly Leu Glu Asn Ser Leu
145                 150                 155                 160

Pro Leu Glu Val Val Arg Gly Ala Met Thr Ile Arg Val Asn Ser Leu
                165                 170                 175

Thr Arg Gly His Ser Ala Val Arg Leu Val Val Leu Glu Ala Leu Thr
            180                 185                 190

Asn Phe Leu Asn His Gly Ile Thr Pro Ile Val Pro Leu Arg Gly Thr
        195                 200                 205

Ile Ser Ala Ser Gly Asp Leu Ser Pro Leu Ser Tyr Ile Ala Ala Ala
    210                 215                 220

Ile Ser Gly His Pro Asp Ser Lys Val His Val Val His Glu Gly Lys
225                 230                 235                 240

Glu Lys Ile Leu Tyr Ala Arg Glu Ala Met Ala Leu Phe Asn Leu Glu
                245                 250                 255

Pro Val Val Leu Gly Pro Lys Glu Gly Leu Gly Leu Val Asn Gly Thr
            260                 265                 270

Ala Val Ser Ala Ser Met Ala Thr Leu Ala Leu His Asp Ala His Met
        275                 280                 285

Leu Ser Leu Leu Ser Gln Ser Leu Thr Ala Met Thr Val Glu Ala Met
    290                 295                 300

Val Gly His Ala Gly Ser Phe His Pro Phe Leu His Asp Val Thr Arg
305                 310                 315                 320

Pro His Pro Thr Gln Ile Glu Val Ala Gly Asn Ile Arg Lys Leu Leu
                325                 330                 335

Glu Gly Ser Arg Phe Ala Val His His Glu Glu Glu Val Lys Val Lys
            340                 345                 350

Asp Asp Glu Gly Ile Leu Arg Gln Asp Arg Tyr Pro Leu Arg Thr Ser
        355                 360                 365

Pro Gln Trp Leu Gly Pro Leu Val Ser Asp Leu Ile His Ala His Ala
    370                 375                 380

Val Leu Thr Ile Glu Ala Gly Gln Ser Thr Thr Asp Asn Pro Leu Ile
385                 390                 395                 400

Asp Val Glu Asn Lys Thr Ser His His Gly Gly Asn Phe Gln Ala Ala
                405                 410                 415

Ala Val Ala Asn Thr Met Glu Lys Thr Arg Leu Gly Leu Ala Gln Ile
            420                 425                 430

Gly Lys Leu Asn Phe Thr Gln Leu Thr Glu Met Leu Asn Ala Gly Met
        435                 440                 445

Asn Arg Gly Leu Pro Ser Cys Leu Ala Ala Glu Asp Pro Ser Leu Ser
    450                 455                 460

Tyr His Cys Lys Gly Leu Asp Ile Ala Ala Ala Ala Tyr Thr Ser Glu
465                 470                 475                 480

Leu Gly His Leu Ala Asn Pro Val Thr Thr His Val Gln Pro Ala Glu
                485                 490                 495

Met Ala Asn Gln Ala Val Asn Ser Leu Ala Leu Ile Ser Ala Arg Arg
            500                 505                 510

Thr Thr Glu Ser Asn Asp Val Leu Ser Leu Leu Leu Ala Thr His Leu
        515                 520                 525

Tyr Cys Val Leu Gln Ala Ile Asp Leu Arg Ala Ile Glu Phe Glu Phe
    530                 535                 540

Lys Lys Gln Phe Gly Pro Ala Ile Val Ser Leu Ile Asp Gln His Phe
545                 550                 555                 560

Gly Ser Ala Met Thr Gly Ser Asn Leu Arg Asp Glu Leu Val Glu Lys
                565                 570                 575

Val Asn Lys Thr Leu Ala Lys Arg Leu Glu Gln Thr Asn Ser Tyr Asp
            580                 585                 590

Leu Val Pro Arg Trp His Asp Ala Phe Ser Phe Ala Ala Gly Thr Val
        595                 600                 605

Val Glu Val Leu Ser Ser Thr Ser Leu Ser Leu Ala Ala Val Asn Ala
    610                 615                 620

Trp Lys Val Ala Ala Ala Glu Ser Ala Ile Ser Leu Thr Arg Gln Val
625                 630                 635                 640

Arg Glu Thr Phe Trp Ser Ala Ala Ser Thr Ser Ser Pro Ala Leu Ser
                645                 650                 655

Tyr Leu Ser Pro Arg Thr Gln Ile Leu Tyr Ala Phe Val Arg Glu Glu
            660                 665                 670

Leu Gly Val Lys Ala Arg Arg Gly Asp Val Phe Leu Gly Lys Gln Glu
        675                 680                 685

Val Thr Ile Gly Ser Asn Val Ser Lys Ile Tyr Glu Ala Ile Lys Ser
    690                 695                 700

Gly Arg Ile Asn Asn Val Leu Leu Lys Met Leu Ala
705                 710                 715

<210> 3
<211> 2151
<212> DNA
<213> Petroselinum crispum

<400> 3
atggagaacg gcaatggtgc aattacaaat ggacatgtaa acgggaacgg aatggatttc 60

tgcatgaaga ccgaagatcc gttgtactgg ggaatcgcgg cggaggcaat gacagggagt 120

catttggatg aagtgaagaa gatggtggct gagtatagga agccggttgt gaagttagga 180

ggagaaacat tgactatttc tcaagtggca gctatttcgg ctagggatgg tagtggtgtc 240

acggtggagc tttccgaggc ggcgagagct ggcgttaagg ctagtagtga ttgggtgatg 300

gatagtatga acaaagggac ggatagttat ggtgtaacta ctggttttgg tgctacttct 360

cataggagga ctaagcaagg tggtgctctt cagaaagagc tcattaggtt cttgaatgct 420

ggaatatttg gtaatggatc agataataca ttaccacact cagcaacaag agctgcaatg 480

ttagtgagga tcaacactct gctccaggga tactctggaa tccgattcga aattctcgaa 540

gcgattacta agtttctaaa ccagaacatt actccttgtt tgccactccg gggtacaatc 600

actgcttctg gtgatcttgt gccattgtct tacattgctg gacttctcac tggccgtccg 660

aattctaagg ctgttggacc tactggagta attcttagcc ccgaagaagc gtttaaactt 720

gctggagtgg aaggaggttt tttcgagttg cagcctaaag aaggccttgc acttgttaat 780

ggaacagctg ttggttctgg tatggcctct atggtacttt tcgaggctaa tatattagct 840

gttttggctg aagttatgtc agcgattttc gctgaagtga tgcaaggaaa gcctgagttc 900

actgaccatt tgacgcataa gttgaagcac catccgggcc aaattgaggc tgcagctata 960

atggaacaca ttttggatgg aagcgcttat gttaaggctg ctcagaagct acatgaaatg 1020

gatccattgc agaagccgaa acaggacaga tatgctctta gaacatctcc tcagtggctt 1080

ggtcctcaga ttgaagtgat tcgatcatcg actaagatga ttgaaagaga gattaactct 1140

gtcaatgata acccgttgat tgatgtttcg aggaacaagg ctattcacgg tggcaatttc 1200

cagggcacac ctattggaat gtcgatggac aatacacgtt tggctattgc cgcgatagga 1260

aagctcatgt ttgctcaatt ttctgaactt gtaaacgact tttacaacaa tgggttgcca 1320

tcaaatttgt ctggagggcg taatccaagt ttggattatg gattcaaggg tgctgaaatt 1380

gcgatggcta gttactgctc tgaacttcaa tttttagcca atccagtgac caaccatgtt 1440

caaagtgctg aacaacacaa tcaagatgta aactccttgg gcttaatctc ttcaaggaaa 1500

acatcagaag ctgttgaaat cttgaaactc atgtctacta catttttagt aggcctctgc 1560

caagcaatag acttgaggca tttggaggag aatttaaaaa gcactgtcaa aaacacagta 1620

agctcagtag ctaagcgagt actaactatg ggtgtcaacg gagaactcca cccctcaaga 1680

ttctgtgaga aagatttact cagatttgtg gaccgcgaat acatttttgc atatattgat 1740

gatccctgca gcgcaaccta cccattgatg caaaaattaa gacaaacact agttgagcat 1800

gcattgaaga atggtgacaa cgagagaaac atgaacactt ccatcttcca aaagattgcg 1860

acctttgagg atgaactaaa ggccctattg cctaaagaag ttgaaagtgc tagagctgca 1920

ctcgaaagtg gaaatccagc aatcccaaac aggatcgagg agtgcaggtc ttacccattg 1980

tacaagtttg tgaggaaaga gttgggaatt gagtatctta caggagaaaa agtaacgtca 2040

cctggagaag agttcgataa ggtatttata gcaatgagca aaggagagat cattgatcca 2100

ttgttagagt gcctggagtc atggaatggt gctcctcttc caatctgtta a 2151

<210> 4
<211> 2151
<212> DNA
<213> Rhodotorola glutinis

<400> 4
atggcaccct cgctcgactc gatctcgcac tcgttcgcaa acggcgtcgc atccgcaaag 60

caggctgtca atggcgcctc gaccaacctc gcagtcgcag gctcgcacct gcccacaacc 120

caggtcacgc aggtcgacat cgtcgagaag atgctcgccg cgccgaccga ctcgacgctc 180

gaactcgacg gctactcgct caacctcgga gacgtcgtct cggccgcgag gaagggcagg 240

cctgtccgcg tcaaggacag cgacgagatc cgctcaaaga ttgacaaatc ggtcgagttc 300

ttgcgctcgc aactctccat gagcgtctac ggcgtcacga ctggatttgg cggatccgca 360

gacacccgca ccgaggacgc catctcgctc cagaaggctc tcctcgagca ccagctctgc 420

ggtgttctcc cttcgtcgtt cgactcgttc cgcctcggcc gcggtctcga gaactcgctt 480

cccctcgagg ttgttcgcgg cgccatgaca atccgcgtca acagcttgac ccgcggccac 540

tcggctgtcc gcctcgtcgt cctcgaggcg ctcaccaact tcctcaacca cggcatcacc 600

cccatcgtcc ccctccgcgg caccatctct gcgtcgggcg acctctctcc tctctcctac 660

attgcagcgg ccatcagcgg tcacccggac agcaaggtgc acgtcgtcca cgagggcaag 720

gagaagatcc tgtacgcccg cgaggcgatg gcgctcttca acctcgagcc cgtcgtcctc 780

ggcccgaagg aaggtctcgg tctcgtcaac ggcaccgccg tctcagcatc gatggccacc 840

ctcgctctgc acgacgcaca catgctctcg ctcctctcgc agtcgctcac ggccatgacg 900

gtcgaagcga tggtcggcca cgccggctcg ttccacccct tccttcacga cgtcacgcgc 960

cctcacccga cgcagatcga agtcgcggga aacatccgca agctcctcga gggaagccgc 1020

tttgctgtcc accatgagga ggaggtcaag gtcaaggacg acgagggcat tctccgccag 1080

gaccgctacc ccttgcgcac gtctcctcag tggctcggcc cgctcgtcag cgacctcatt 1140

cacgcccacg ccgtcctcac catcgaggcc ggccagtcga cgaccgacaa ccctctcatc 1200

gacgtcgaga acaagacttc gcaccacggc ggcaatttcc aggctgccgc tgtggccaac 1260

accatggaga agactcgcct cgggctcgcc cagatcggca agctcaactt cacgcagctc 1320

accgagatgc tcaacgccgg catgaaccgc ggcctcccct cctgcctcgc ggccgaagac 1380

ccctcgctct cctaccactg caagggcctc gacatcgccg ctgcggcgta cacctcggag 1440

ttgggacacc tcgccaaccc tgtgacgacg catgtccagc cggctgagat ggcgaaccag 1500

gcggtcaact cgcttgcgct catctcggct cgtcgcacga ccgagtccaa cgacgtcctt 1560

tctctcctcc tcgccaccca cctctactgc gttctccaag ccatcgactt gcgcgcgatc 1620

gagttcgagt tcaagaagca gttcggccca gccatcgtct cgctcatcga ccagcacttt 1680

ggctccgcca tgaccggctc gaacctgcgc gacgagctcg tcgagaaggt gaacaagacg 1740

ctcgccaagc gcctcgagca gaccaactcg tacgacctcg tcccgcgctg gcacgacgcc 1800

ttctccttcg ccgccggcac cgtcgtcgag gtcctctcgt cgacgtcgct ctcgctcgcc 1860

gccgtcaacg cctggaaggt cgccgccgcc gagtcggcca tctcgctcac ccgccaagtc 1920

cgcgagacct tctggtccgc cgcgtcgacc tcgtcgcccg cgctctcgta cctctcgccg 1980

cgcactcaga tcctctacgc cttcgtccgc gaggagcttg gcgtcaaggc ccgccgcgga 2040

gacgtcttcc tcggcaagca agaggtgacg atcggctcga acgtctccaa gatctacgag 2100

gccatcaagt cgggcaggat caacaacgtc ctcctcaaga tgctcgctta g 2151

<210> 5
<211> 5711
<212> DNA
<213> Artificial

<220>
<223> Vector

<400> 5
ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc cggatatagt tcctcctttc agcaaaaaac ccctcaagac ccgtttagag 240

gccccaaggg gttatgctag ttattgctca gcggtggcag cagccaactc agcttccttt 300

cgggctttgt tagcagccgg atcctcgagc atatgacgac cttcgatatg gccgctgctg 360

tgatgatgat gatgatgatg atgatgatgg cccatggtat atctccttct taaagttaaa 420

caaaattatt tctagagggg aattgttatc cgctcacaat tcccctatag tgagtcgtat 480

taatttcgcg ggatcgagat ctcgatcctc tacgccggac gcatcgtggc cggcatcacc 540

ggcgccacag gtgcggttgc tggcgcctat atcgccgaca tcaccgatgg ggaagatcgg 600

gctcgccact tcgggctcat gagcgcttgt ttcggcgtgg gtatggtggc aggccccgtg 660

gccgggggac tgttgggcgc catctccttg catgcaccat tccttgcggc ggcggtgctc 720

aacggcctca acctactact gggctgcttc ctaatgcagg agtcgcataa gggagagcgt 780

cgagatcccg gacaccatcg aatggcgcaa aacctttcgc ggtatggcat gatagcgccc 840

ggaagagagt caattcaggg tggtgaatgt gaaaccagta acgttatacg atgtcgcaga 900

gtatgccggt gtctcttatc agaccgtttc ccgcgtggtg aaccaggcca gccacgtttc 960

tgcgaaaacg cgggaaaaag tggaagcggc gatggcggag ctgaattaca ttcccaaccg 1020

cgtggcacaa caactggcgg gcaaacagtc gttgctgatt ggcgttgcca cctccagtct 1080

ggccctgcac gcgccgtcgc aaattgtcgc ggcgattaaa tctcgcgccg atcaactggg 1140

tgccagcgtg gtggtgtcga tggtagaacg aagcggcgtc gaagcctgta aagcggcggt 1200

gcacaatctt ctcgcgcaac gcgtcagtgg gctgatcatt aactatccgc tggatgacca 1260

ggatgccatt gctgtggaag ctgcctgcac taatgttccg gcgttatttc ttgatgtctc 1320

tgaccagaca cccatcaaca gtattatttt ctcccatgaa gacggtacgc gactgggcgt 1380

ggagcatctg gtcgcattgg gtcaccagca aatcgcgctg ttagcgggcc cattaagttc 1440

tgtctcggcg cgtctgcgtc tggctggctg gcataaatat ctcactcgca atcaaattca 1500

gccgatagcg gaacgggaag gcgactggag tgccatgtcc ggttttcaac aaaccatgca 1560

aatgctgaat gagggcatcg ttcccactgc gatgctggtt gccaacgatc agatggcgct 1620

gggcgcaatg cgcgccatta ccgagtccgg gctgcgcgtt ggtgcggata tctcggtagt 1680

gggatacgac gataccgaag acagctcatg ttatatcccg ccgttaacca ccatcaaaca 1740

ggattttcgc ctgctggggc aaaccagcgt ggaccgcttg ctgcaactct ctcagggcca 1800

ggcggtgaag ggcaatcagc tgttgcccgt ctcactggtg aaaagaaaaa ccaccctggc 1860

gcccaatacg caaaccgcct ctccccgcgc gttggccgat tcattaatgc agctggcacg 1920

acaggtttcc cgactggaaa gcgggcagtg agcgcaacgc aattaatgta agttagctca 1980
ctcattaggc accgggatct cgaccgatgc ccttgagagc cttcaaccca gtcagctcct 2040
tccggtgggc gcggggcatg actatcgtcg ccgcacttat gactgtcttc tttatcatgc 2100
aactcgtagg acaggtgccg gcagcgctct gggtcatttt cggcgaggac cgctttcgct 2160
ggagcgcgac gatgatcggc ctgtcgcttg cggtattcgg aatcttgcac gccctcgctc 2220
aagccttcgt cactggtccc gccaccaaac gtttcggcga gaagcaggcc attatcgccg 2280
gcatggcggc cgacgcgctg ggctacgtct tgctggcgtt cgcgacgcga ggctggatgg 2340
ccttccccat tatgattctt ctcgcttccg gcggcatcgg gatgcccgcg ttgcaggcca 2400
tgctgtccag gcaggtagat gacgaccatc agggacagct tcaaggatcg ctcgcggctc 2460
ttaccagcct aacttcgatc actggaccgc tgatcgtcac ggcgatttat gccgcctcgg 2520
cgagcacatg gaacgggttg gcatggattg taggcgccgc cctatacctt gtctgcctcc 2580
ccgcgttgcg tcgcggtgca tggagccggg ccacctcgac ctgaatggaa gccggcggca 2640
cctcgctaac ggattcacca ctccaagaat tggagccaat caattcttgc ggagaactgt 2700
gaatgcgcaa accaaccctt ggcagaacat atccatcgcg tccgccatct ccagcagccg 2760
cacgcggcgc atctcgggca gcgttgggtc ctggccacgg gtgcgcatga tcgtgctcct 2820
gtcgttgagg acccggctag gctggcgggg ttgccttact ggttagcaga atgaatcacc 2880
gatacgcgag cgaacgtgaa gcgactgctg ctgcaaaacg tctgcgacct gagcaacaac 2940
atgaatggtc ttcggtttcc gtgtttcgta aagtctggaa acgcggaagt cagcgccctg 3000
caccattatg ttccggatct gcatcgcagg atgctgctgg ctaccctgtg gaacacctac 3060
atctgtatta acgaagcgct ggcattgacc ctgagtgatt tttctctggt cccgccgcat 3120
ccataccgcc agttgtttac cctcacaacg ttccagtaac cgggcatgtt catcatcagt 3180
aacccgtatc gtgagcatcc tctctcgttt catcggtatc attaccccca tgaacagaaa 3240
tcccccttac acggaggcat cagtgaccaa acaggaaaaa accgccctta acatggcccg 3300
ctttatcaga agccagacat taacgcttct ggagaaactc aacgagctgg acgcggatga 3360
acaggcagac atctgtgaat cgcttcacga ccacgctgat gagctttacc gcagctgcct 3420
cgcgcgtttc ggtgatgacg gtgaaaacct ctgacacatg cagctcccgg agacggtcac 3480
agcttgtctg taagcggatg ccgggagcag acaagcccgt cagggcgcgt cagcgggtgt 3540
tggcgggtgt cggggcgcag ccatgaccca gtcacgtagc gatagcggag tgtatactgg 3600
cttaactatg cggcatcaga gcagattgta ctgagagtgc accatatatg cggtgtgaaa 3660
taccgcacag atgcgtaagg agaaaatacc gcatcaggcg ctcttccgct tcctcgctca 3720
ctgactcgct gcgctcggtc gttcggctgc ggcgagcggt atcagctcac tcaaaggcgg 3780
taatacggtt atccacagaa tcaggggata acgcaggaaa gaacatgtga gcaaaaggcc 3840
agcaaaaggc caggaaccgt aaaaaggccg cgttgctggc gtttttccat aggctccgcc 3900
cccctgacga gcatcacaaa aatcgacgct caagtcagag gtggcgaaac ccgacaggac 3960
tataaagata ccaggcgttt ccccctggaa gctccctcgt gcgctctcct gttccgaccc 4020
tgccgcttac cggatacctg tccgcctttc tcccttcggg aagcgtggcg ctttctcata 4080
gctcacgctg taggtatctc agttcggtgt aggtcgttcg ctccaagctg ggctgtgtgc 4140
acgaaccccc cgttcagccc gaccgctgcg ccttatccgg taactatcgt cttgagtcca 4200

acccggtaag acacgactta tcgccactgg cagcagccac tggtaacagg attagcagag 4260
cgaggtatgt aggcggtgct acagagttct tgaagtggtg gcctaactac ggctacacta 4320
gaaggacagt atttggtatc tgcgctctgc tgaagccagt taccttcgga aaaagagttg 4380
gtagctcttg atccggcaaa caaaccaccg ctggtagcgg tggttttttt gtttgcaagc 4440
agcagattac gcgcagaaaa aaaggatctc aagaagatcc tttgatcttt tctacggggt 4500
ctgacgctca gtggaacgaa aactcacgtt aagggatttt ggtcatgaga ttatcaaaaa 4560
ggatcttcac ctagatcctt ttaaattaaa aatgaagttt taaatcaatc taaagtatat 4620
atgagtaaac ttggtctgac agttaccaat gcttaatcag tgaggcacct atctcagcga 4680
tctgtctatt tcgttcatcc atagttgcct gactccccgt cgtgtagata actacgatac 4740
gggagggctt accatctggc cccagtgctg caatgatacc gcgagaccca cgctcaccgg 4800
ctccagattt atcagcaata aaccagccag ccggaagggc cgagcgcaga agtggtcctg 4860
caactttatc cgcctccatc cagtctatta attgttgccg ggaagctaga gtaagtagtt 4920
cgccagttaa tagtttgcgc aacgttgttg ccattgctgc aggcatcgtg gtgtcacgct 4980
cgtcgtttgg tatggcttca ttcagctccg gttcccaacg atcaaggcga gttacatgat 5040
cccccatgtt gtgcaaaaaa gcggttagct ccttcggtcc tccgatcgtt gtcagaagta 5100
agttggccgc agtgttatca ctcatggtta tggcagcact gcataattct cttactgtca 5160
tgccatccgt aagatgcttt tctgtgactg gtgagtactc aaccaagtca ttctgagaat 5220
agtgtatgcg gcgaccgagt tgctcttgcc cggcgtcaac acgggataat accgcgccac 5280
atagcagaac tttaaaagtg ctcatcattg gaaaacgttc ttcggggcga aaactctcaa 5340
ggatcttacc gctgttgaga tccagttcga tgtaacccac tcgtgcaccc aactgatctt 5400
cagcatcttt tactttcacc agcgtttctg ggtgagcaaa aacaggaagg caaaatgccg 5460
caaaaaaggg aataagggcg acacggaaat gttgaatact catactcttc ctttttcaat 5520
attattgaag catttatcag ggttattgtc tcatgagcgg atacatattt gaatgtattt 5580
agaaaaataa acaaataggg gttccgcgca catttccccg aaaagtgcca cctgacgtct 5640
aagaaaccat tattatcatg acattaacct ataaaaatag gcgtatcacg aggccctttc 5700
gtcttcaaga a 5711